# FASCICULE DE BREVET EUROPEEN

(11) **EP 1 347 950 B1**
(45) Date de publication et mention de la délivrance du brevet: **17.02.2010**
(21) Numéro de dépôt: 01997471.6
(22) Date de dépôt: 16.11.2001
(51) Int. Cl.: C07C 59/48

(54) **DERIVES DE L'ACIDE 4-HYDROXYBUTANOIQUE ET DE SON HOMOLOGUE SUPERIEUR COMME LIGANDS DES RECEPTEURS DU GAMMA-HYDROXYBUTYRATE (GHB) COMPOSITIONS PHARMACEUTIQUES LES CONTENANT ET UTILISATIONS PHARMACEUTIQUES**
DERIVATE VON 4-HYDROXYBUTANSÄURE UND DEREN HÖHEREN HOMOLOGEN ALS LIGANDEN VON G-HYDROXYBUTYRAT- (GBH-)REZEPTOREN, PHARMAZEUTISCHE ZUSAMMENSETZUNGEN, DIE DIESE ENTHALTEN, UND PHARMAZEUTISCHE ANWENDUNGEN
DERIVATIVES OF 4-HYDROXYBUTANOIC ACID AND OF ITS HIGHER HOMOLOGUE AS LIGANDS OF GAMMA-HYDROXYBUTYRATE (GHB) RECEPTORS, PHARMACEUTICAL COMPOSITIONS CONTAINING SAME AND PHARMACEUTICAL USES

(30) Priorité: 27.11.2000 FR 0015291
(43) Date de publication de la demande: 01.10.2003
(73) Titulaire: Université Louis Pasteur (Etablissement Public à Caractère Scientifique, Culturel et Professionnel), 67000 Strasbourg (FR)
(72) Inventeur: BOURGUIGNON, Jean-Jacques, F-67150 Hipsheim (FR); MAITRE, Michel, F-67000 Strasbourg (FR); KLOTZ, Evelyne, F-67190 Mutzig (FR); SCHMITT, Martine, F-67000 Strasbourg (FR); GOBAILLE, Serge, F-67550 Vendenheim (FR); MACHER, Jean-Paul, F-68500 Bergholtz-Zell (FR)
(74) Mandataire: Nuss, Pierre
(86) Numéro de dépôt international: PCT/FR2001/003615
(87) Numéro de publication internationale: WO 2002/042250

(56) Documents cités:
- EP-A- 0 252 639
- EP-A- 0 295 050
- WO-A-95/13262
- DE-A- 2 112 716
- DE-A- 2 141 926
- DE-A- 4 009 117
- FR-A- 2 279 385
- GB-A- 1 388 146
- US-A- 3 859 338
- US-A- 4 948 809
- US-A- 5 451 578
- BOURGUIGNON, JEAN JACQUES ET AL: "Analogs of.gamma.-hydroxybutyric acid. Synthesis and binding studies" J. MED. CHEM. (1988), 31(5), 893-7, XP002177958
- CLAWSON, P. ET AL: "Synthetic Studies on O-Heterocycles via Cycloadditions. Part 2. Adducts from Styrene Oxides" JOURNAL OF THE CHEMICAL SOCIETY, PERKING TRANSACTIONS 1, 1990, pages 159-162, XP002177961
- YOUNG, R. N. ET AL: "Design and Synthesis of Sodium-4-((3-(4-Acetyl-3-hydroxy-2-propyl- phenoxy)propyl)thio)-gamma-hydroxy-beta-me thyl-benzenebutanoate: A Novel, Selective, and Orally Active Receptor Antagonist of Leukotriene D4" JOURNAL OF MEDICINAL CHEMISTRY, vol. 29, 1986, pages 1573-1576, XP002177962
- BENNANI, Y AND SHARPLESS, K.B.: "Asymmetric Synthesis of gamma-hydroxy alpha, beta-unsaturated amides via an AD-elimination process; Synthesi of (+)-Coriolic acid" TETRAHEDRON LETTERS, vol. 34, no. 13, 1993, pages 2083-2086, XP002177975
- ASTLES, PETER C. ET AL: "Selective Endothelin A Receptor Antagonists. 3. Discovery and Structure-Activity Relationships of a Series of 4-Phenoxybutanoic Acid Derivatives" J. MED. CHEM. (1998), 41(15), 2732-2744, XP002177959
- DJURIC, STEVAN W. ET AL: "Synthesis of 5-fluoroprostacyclin" J. ORG. CHEM. (1987), 52(6), 978-90, XP002177960

## Description

La présente invention concerne le domaine de la chimie organique de synthèse appliquée au domaine pharmaceutique et a pour objet de nouveaux dérivés de l'acide 4-hydroxybutanoïque et son homologue supérieur, l'acide 5-hydroxypentanoïque, de leurs homologues crotoniques, les compositions pharmaceutiques les contenant ainsi que leurs utilisations pharmaceutiques. Ces nouveaux dérivés sont susceptibles de se lier aux récepteurs spécifiques du γ-hydroxybutyrate (GHB) et sont susceptibles de présenter de ce fait des propriétés agonistes ou antagonistes.

La présente invention concerne également des esters de ces composés possédant une efficacité thérapeutique orale améliorée. Enfin, la présente invention concerne également les utilisations spécifiques de ces GHB-mimétiques, notamment dans le traitement des perturbations du sommeil, de l'anxiété et des affections générales du système nerveux central (SNC).

Le γ-hydroxybutyrate a été étudié dès 1960 par LABORIT comme analogue isostère de l'acide γ-aminobutyrique (GABA) susceptible de passer la barrière hématoencéphalique. En fait, le GHB a été identifié comme un composant naturel du cerveau des mammifères et est actuellement utilisé en thérapeutique comme anesthésique général bien toléré.

Son catabolisme est très rapide, le temps de demi-vie étant, à titre indicatif, d'environ une heure chez le chat. Le GHB induit une sédation profonde avec pertes de réflexes de posture et analgésie. Le tracé électroencéphalographique a été interprété tantôt chez certaines espèces animales comme un tracé de sommeil sans perturbation de sommeil paradoxal, tantôt comme un tracé rappelant celui du « petit mal » épileptique. Ce dernier inconvénient a réduit les indications du GHB à l'anesthésie chez les personnes âgées.

On a pu démontrer dans les années 1980 que le GHB remplissait les principaux critères qui sont généralement retenus pour la qualification d'une substance en tant que neurotransmetteur ou neuromodulateur, à savoir, présence de l'enzyme de synthèse de ce composé dans des terminaisons présynaptiques, libération calcium-dépendante par la dépolarisation membranaire, transport actif de haute affinité sodium dépendant et enfin fixation réversible saturable de haute affinité sur des préparations membranaires d'origine synaptique.

Toutes ces propriétés permettent d'établir l'existence de circuits neuronaux dans lesquels un rôle du γ-hydroxybutyrate est directement impliqué.

Le document EP 0 295 050 A décrit des dérivés de la sertraline et leur activité en tant qu'anti-dépresseurs. L'acide 4-(3,4-dichlorophényl)-4-hydroxybutyrique qui y est cité n'est utilisé qu'en tant qu'intermédiaire de synthèse.

Le document FR 2 279 385 A décrit la synthèse de dérivés de l'acide 4-(4-biphénylyl)-butyrique ainsi que leur activité antiphlogistique et inhibiteur de prolifération.

Le brevet US 4 498 809 A décrit des sulfonamides ayant un substituant « phényl-GHB » et revendique leur activité anti-aggrégant plaquettaire. Il ne décrit pas de molécules qui font partie des molécules revendiquées dans la présente demande, ni d'un point de vue structurel, ni du point de vue de leurs activités pharmacologiques.

La demande WO 95 13262 A décrit la synthèse de composés de type « phényl » diversement substitués et revendique leur activité inhibitrice d'endothéline. L'acide 4-(2-méthylphényl)-4-hydroxybutyrique cité y est utilisé comme intermédiaire de synthèse.

Le document GB 1 388 146 A décrit des dérivés de l'acide 4-hydroxycrotonique et leurs activités anti-inflammatoires. Toutefois, ce document ne mentionne ni ne suggère une quelconque activité biologique de ces composés au niveau du système nerveux central en agissant sur le récepteur GHB (gamma-hydroxy-butyrate).

La demande européenne EP 0 252 639 A décrit des dérivés le leucotriènes ayant pour quelques exemples un reste GHB avec un groupement γ-phényle substitué par un groupement thioalkyle. Les composés de la présente invention ne concernent pas ces structures moléculaires ni leur utilisation pour une activité anti-inflammatoire.

La publication XP 002177958 décrit la synthèse de certains dérivés du GHB et leur activité in vitro de liaison au récepteur du GHB.

Le document DE 40 09 117 A décrit des dérivés de leucotriènes ayant pour quelques exemples un reste GHB avec un groupement γ-phényle substitué par une chaîne grasse insaturée en C10 ou γ-pyridyle substitué par une chaîne grasse insaturée en C10. Les composés ne concernent pas non plus les structures moléculaires ni les utilisations de la présente invention.

Le brevet US 5 451 578 A décrit entre autres des dérivés de l'acide valérique substitués en C5 par des bicycles spiro-azotés et leur activité en tant qu'anti-aggrégant plaquettaire.

Les composés de la présente invention ne concernent pas non plus ces structures moléculaires ni leur utilisation pour cette activité biologique particulière.

La publication XP 002177961 décrit la synthèse de quatre dérivés de l'acide 4-hydroxycrotonique comme intermédiaires de synthèse d'hétérocycles oxygénés via une cycloaddition. Aucune activité biologique n'y est cependant décrite.

A l'instar de la demande EP 0 252 639 A, la publication XP 002177962 décrit des dérivés de leucotriènes ayant pour quelques exemples un reste GHB avec un groupement γ-phényle substitué par un thioalkyle. De plus, les produits finaux cités portent un groupement méthyle en position β de la chaîne GHB. Les composés de la présente invention ne concernent pas non plus ces structures moléculaires ni leur utilisation pour une activité anti-inflammatoire.

Les documents DE 21 41 926 A, DE 21 12 716 A et US 3 859 338 A décrivent, quant à eux, des dérivés 4-(4-biphénylyl)-GHB.

La publication XP 002177975 décrit une méthode de synthèse permettant d'aboutir de façon énantioselective à un dérivé amide d'acide 4-phényl-4-hydroxycrotonique.

La publication XP 002177959 décrit des dérivés d'acide 4-phénoxybutyrique pour leur activité antagoniste des récepteurs à l'endothéline A. L'acide 4-(2-méthylphényl)-4-hydroxybutyrique y est utilisé comme intermédiaire de synthèse.

La publication XP 002177960 décrit la synthèse de la 5-fluoroprostacycline. Aucune des molécules décrites ne tombe dans les revendications de la présente demande de brevet.

En résumé, aucun des documents précités ne décrit les composés γ-aryl-GHB bi ou tricycliques de la présente invention. Seuls quelques molécules simples de γ-aryl-GHB différemment substitués sont divulguées et généralement uniquement en tant qu'intermédiaires de synthèse chimique. De plus, aucune des publications précitées ne décrit ou ne suggère une quelconque activité centrale de type GHB in vivo.

La présente invention a pour but de proposer de nouveaux dérivés de l'acide 4-hydroxybutanoïque, de l'acide 5-hydroxypentanoïque et de leurs dérivés crotoniques qui sont susceptibles de se lier plus efficacement aux récepteurs spécifiques du γ-hydroxybutyrate (GHB) et qui présentent des propriétés agonistes ou antagonistes.

De manière inattendue, les inventeurs ont mis en évidence qu'en fonctionnalisant des composés de la famille de l'acide 4-hydroxybutanoïque ou de l'acide 5-hydroxypentanoïque (l'acide, ses esters, ses sels sodiques, ses dérivés crotoniques...), en particulier par substitution aromatique du carbone méthylique en bout de chaîne, il était possible d'obtenir des dérivés synthétiques présentant une augmentation de l'affinité pour les récepteurs du GHB par rapport à l'affinité du GHB naturel pour ces mêmes récepteurs.

Dans un premier aspect, la présente invention a pour objet les composés de la revendication 1.

Plus préférentiellement, elle a pour objet les composés de la revendication 2.

En particulier elle a pour objet les sels de formule générale I" 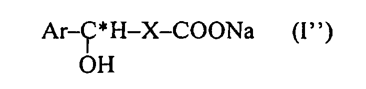 dans laquelle Ar et X sont tels que définis aux revendications 1 ou 2, qui sont obtenus à partir de ces dernières et qui peuvent être préparés selon les techniques connues de l'homme du métier.

Dans un second aspect, la présente invention fournit des compositions pharmaceutiques comprenant à titre de principe actif au moins un composé de formule générale I, I' ou I" telles que définies aux revendications 1 à 3. Elle a également pour objet l'utilisation à des fins thérapeutiques des nouveaux dérivés synthétisés telle que définie à la revendication 26.

La présente invention décrit différentes voies de synthèses, qui se rapportent à des modes de réalisations préférés, donnés à titre d'exemples non limitatifs, et expliqués avec référence aux dessins schématiques annexés, dans lesquels :
la figure 1 représente un schéma synoptique simplifié des différents procédés de synthèse des composés selon l'invention ainsi que de leurs principaux intermédiaires réactionnels,
la figure 2 représente un schéma simplifié d'autres procédés de synthèse de composés selon l'invention,
la figure 3 représente un autre schéma simplifié d'autres procédés de synthèse de composés selon l'invention ; et,

Sauf indication contraire, les termes suivants utilisés dans la présente description et le présent jeu de revendications ont les significations suivantes :
- « alkyle » désigne un radical hydrocarbure monovalent saturé linéaire ayant de 1 à 6 atomes de carbone ou un radical hydrocarbure monovalent saturé ramifié ayant de 3 à 6 atomes de carbone, par exemple, méthyle, éthyle, propyle, 2-propyle, butyle, etc.
- « aryle » désigne un radical hydrocarbure aromatique de type phényle éventuellement substitué, c'est-à-dire un groupe phényle qui est éventuellement substitué indépendamment avec un ou deux substituants choisis parmi le groupe formé par les groupements halogéno, -OR (où R représente un atome d'hydrogène ou un groupe alkyle) et -NRR' (où R et R' représentent, indépendamment l'un de l'autre, un atome d'hydrogène ou un groupe alkyle).
- « aralkyle » désigne un radical aryle défini ci-dessus substitué par un groupement alkylène, c'est-à-dire, par un radical hydrocarbure divalent saturé linéaire de 1 à 6 atomes de carbone ou un radical hydrocarbure divalent saturé ramifié de 3 à 6 atomes de carbone contenant au moins une liaison double, tels que benzyle, phényléthyle, etc.
- « halogène » désigne un radical monovalent choisi parmi le fluor, le chlore, le brome et l'iode.

Les expressions « atome de carbone » et « atome d'azote » doivent être comprises comme désignant lesdits atomes, éventuellement pourvus d'un ou de deux atomes d'hydrogène et/ou substitués par les groupements précités et/ou comportant une liaison chimique, de façon à satisfaire les valences habituelles du carbone et de l'azote. A titre d'exemples, z peut représenter, selon le cas, N, NH, C, CH ou CH₂, Y peut être, selon le cas, C, CH, CH₂, S, 0...

Les composés selon la présente invention peuvent posséder un ou plusieurs centres asymétriques et peuvent donc être produits en tant que stéréoisomères (R) ou (S) individuels ou en tant que mélanges de ceux-ci. Sauf indication particulière, la présente description et le présent jeu de revendications englobent à la fois les énantiomères individuels et leurs mélanges en toutes proportions, y compris les mélanges dits « racémiques ».

Par « excipient acceptable sur le plan pharmaceutique » l'on désigne un seul ou un ensemble de plusieurs excipients qui sont utiles dans la préparation d'une composition pharmaceutique qui est généralement sûre, non toxique et ni biologiquement ni autrement non souhaitable, et inclut un ou des excipients qui sont acceptables aussi bien pour une utilisation vétérinaire que pour une utilisation pharmaceutique humaine.

Par « sel acceptable sur le plan pharmaceutique » l'on désigne un sel ou un mélange de plusieurs sels qui sont utiles dans la préparation d'une composition pharmaceutique qui est généralement sûre, non toxique et ni biologiquement ni autrement non souhaitable et qui possède l'activité pharmacologique souhaitée de la molécule mère. De tels sels incluent les sels formés quand un proton acide de la molécule mère est remplacé par un ion métal, par exemple, un ion de métal alcalin (de préférence le sodium ou le lithium) ou un ion alcalino-terreux, soit se coordonne avec une base organique telle que l'éthanolamine, la diéthanolamine, les acides aminés (en particulier la lysine), etc.

Un « contre-ion acceptable sur le plan pharmaceutique » désigne un ion ayant une charge opposée à celle de la substance avec laquelle il est associé et qui est acceptable sur le plan pharmaceutique.

Le terme esters inclut par exemple, les dérivés d'acétate, de formiate, de benzoate etc.

Les termes « traiter » ou « traitement » d'une maladie incluent :
- d'empêcher la maladie, à savoir amener les symptômes cliniques de la maladie à ne pas se développer chez un mammifère qui peut être exposé ou prédisposé à la maladie mais qui n'éprouve ou qui ne présente pas encore de symptômes de la maladie,
- d'inhiber la maladie, à savoir, arrêter ou réduire le développement de la maladie ou de ses symptômes cliniques, ou
- de faire disparaître la maladie, à savoir, entraîner la régression de la maladie ou de ses symptômes cliniques.

Une « quantité efficace sur le plan thérapeutique » désigne la quantité d'un composé qui, lorsque administrée à un mammifère pour traiter une maladie, est suffisante pour effectuer un tel traitement pour la maladie. Cette quantité variera en fonction du composé, de la maladie et de sa gravité, de l'âge, du poids, etc., du mammifère à traiter.

Les composés de départ peuvent être obtenus dans le commerce ou synthétisés selon les procédés habituels. Il est entendu que la présente demande n'est pas limitée à une voie de synthèse particulière, et s'étend à d'autres procédés permettant l'élaboration des composés indiqués.

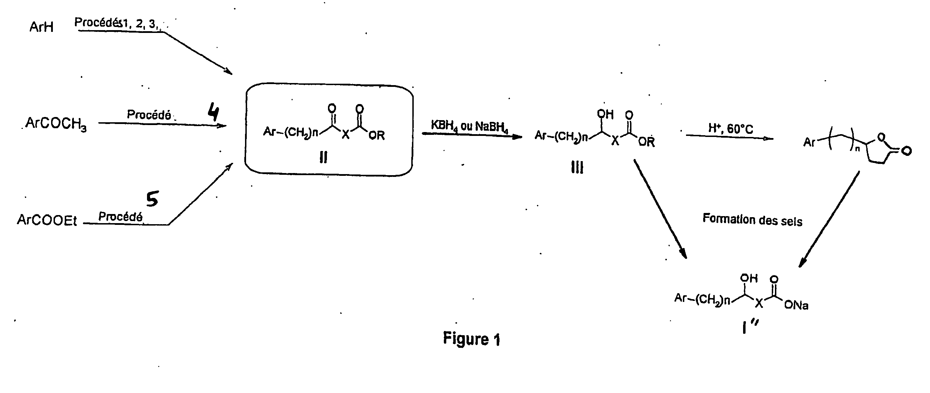

Les composés de formule générale I" sont préparés comme illustré dans le schéma de la figure 1. Les intermédiaires clés sont les acides de formule générale I et les céto-acides ou esters correspondants de formule générale II :

Ar-(C*=O)-X-CO₂R (II)

où R peut être l'hydrogène ou un groupement alkyle, de préférence éthyle ou méthyle.

Suivant la nature de l'aromatique (Ar), ces dérivés sont obtenus soit par réaction de Friedel-Crafts (M. Kakushima et al. JOC, 48(19), 1983, 3214 ; G. J. Quallich et al. JOC, 55(16), 1990, 4971) soit par condensation d'une méthylcétone avec l'acide glyoxylique (J. J. Bourguignon et al. J. Med. Chem., 1988, 31, 893-897) soit par action de ArCO₂Et sur le diéthylsuccinate en milieu basique (H. Michel et al. Arch. Pharmaz., 1974, 307, 689). Des exemples de réalisation seront donnés dans la partie A) ci-après (cf. notamment le tableau I de cette partie). Ces céto-acides ou esters II sont par la suite réduits en alcools correspondants de formule générale III à l'aide de NaBH₄ dans le cas où R=Me ou R=Et et par KBH₄ si R=H, puis salifié à l'aide de NaOH pour obtenir les sels de sodium correspondants de formule générale I", comme représenté sur la figure 1.

Comme également indiqué sur le schéma de la figure 1, les composés réduits III se lactonisent en composés de formule générale IV par simple chauffage en milieu acide. Cette réaction est bien connue de l'homme du métier et ne nécessite pas d'être développée plus en détail ici. Bien entendu, l'homme du métier pourra choisir tout acide adapté pour obtenir les lactones souhaitées. A titre d'exemple d'acide particulièrement adapté on peut citer, comme illustré de manière non-limitative dans les exemples qui suivent, l'acide chlorhydrique. L'action d'une amine sur les lactones IV conduit aux amides correspondants de formule générale V.

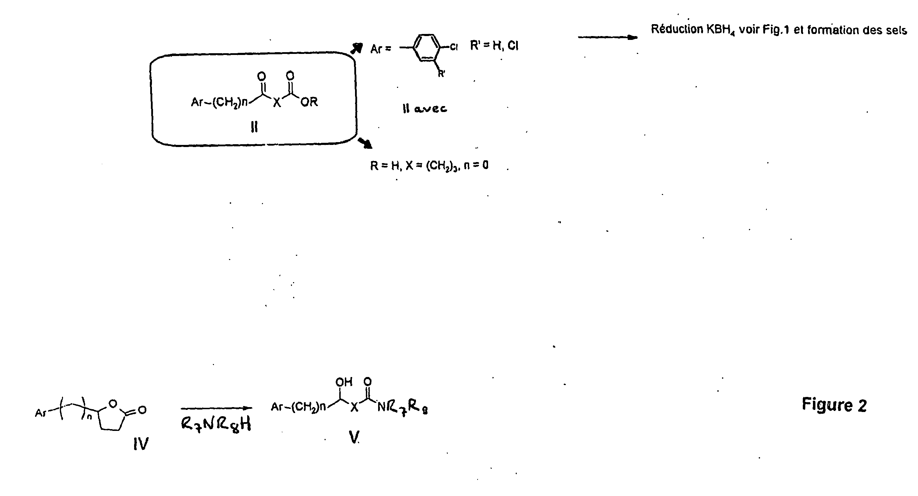

Par ailleurs, les céto-acides pour lesquels R=H, X=(CH₂)₃ et n=0 peuvent être transformés en γ-aroyl-γ-butyro-lactones de formule générale VI par réaction avec le brome (K. Yamada et al., Tetrahedron, 1971, 27, 5445-5451). Ces lactones VI peuvent ensuite être réduites en γ-benzyl-lactones de formule générale IV par hydrogénation catalytique. Cependant, ce dernier procédé n'est pas applicable si l'aromatique Ar porte un halogène. Dans ce cas, on fait appel à une réaction catalysée par le Pd(OAc)₄. Ainsi, en traitant, par exemple, le 1-chloro-4-iodobenzène on obtient la méthylidine-γ-butyrolactone de formule générale VII (A. Arcadi et al. JOC, 1992, 57, 976-982) qui par action de NaOH conduit au céto-acide II correspondant (cf. figure 2).

I1 sera à présent décrit plus en détail, à l'aide d'exemples illustratifs non limitatifs, les différentes étapes précitées de synthèse des composés selon l'invention.

### A) Accès aux céto-acides ou céto-esters intermédiaires (II)

La formation des céto-acides ou céto-esters intermédiaires de formule générale II est décrite ci-après, les différents procédés d'obtention étant résumés dans le tableau 1 suivant.

**Tableau 1 : Procédés d'obtention des céto-acides ou céto-esters intermédiaires**

| Réactifs de départ | | X | n | R | Procédé n° |
|---|---|---|---|---|---|
| ArH | 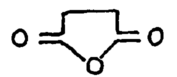 | (CH₂)₂ | 0 | H | 1 |
| ArH | 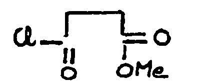 | (CH₂)₂ | 0 | Me | 2 |
| ArH | 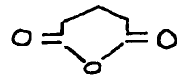 | (CH₂)₃ | 0 | H | 3 |
| ArCOCH₃ | CHO-CO₂H | (CH₂)₂ | 0 | H | 4 |
| ArCO₂Et | 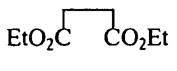 | (CH₂)₂ | 0 | H | 5 |

Cinq procédés et onze exemples de modes opératoires seront détaillés dans ce qui suit. Les céto-esters obtenus sont réduits en lactones et salifiés comme indiqué sur le schéma de la figure 1.

### 1) Procédé 1 : réaction de Friedel et Crafts avec l'anhydride succinique

Deux exemples précisant les conditions opératoires de solvants, température et temps de réaction sont décrits ci-après.

### Exemple 1 : synthèse de l'acide 4-(2,3-dihydro-1H-inden-5-yl)-4-oxobutanoïque (dérivé céto-acide du composé N°15)

On effectue une réaction de Friedel et Crafts classique dans le nitrobenzène (60 ml) à température ambiante pendant 4 heures : indane (4 ml, 32,66 mmoles), AlCl₃ (11,00 g, 82,5 mmoles) et anhydride succinique (3,00 g, 30 mmoles). On obtient un solide légèrement jaune caractérisé comme suit :

| | |
|---|---|
| solide légèrement jaune | 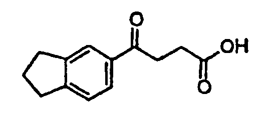 |
| MM (C₁₃H₁₄O₃): 218,25 g | |
| masse : 5,03 g | |
| Rdt. : 71% | |

RMN (¹H, 200 MHz, DMSO) : 12,13 (large s, 1H, -COOH) ; 7,84 (s, 1H, CHarom.) ; 7,78 (d, 1H, CHarom., J³ = 7,8 Hz) ; 7,37 (d, 1H, CHarom., J³ = 7,8 Hz) ; 3,23 (t, 2H, -CH₂-, J³ = 5,9 Hz) ; 2,93 (m, 4H, 2-CH₂-) ; 2,57 (t, 2H, -CH₂-, J³ = 5,9 Hz) ; 2,05 (m, 2H, -CH₂-).

### Exemple 2 : synthèse de l'acide 4-(5-acétyl-10,11-dihydro-5H-dibenzo[b,f]azepin-3-yl)-4-oxobutanoïque (dérivé céto-acide du composé N° 30)

Le dérivé N-acétylé d'azépine (1,00 g, 4,21 mmoles) est dissout dans CS₂ (10 ml) et le chlorure d'aluminium (5,62 g, 42,1 mmoles) est ajouté. L'anhydride succinique (3,37 g, 33,8 mmoles) est ensuite ajouté par petites spatules. Le milieu réactionnel est agité (agitation mécanique) à reflux pendant 6 heures. Après refroidissement, cette solution est versée sur de la glace et extraite à l'acétate d'éthyle. La phase organique est traitée avec NaOH 1N et la phase aqueuse lavée avec l'acétate d'éthyle. Puis, on repasse en milieu acide par addition de HCl concentré. Le produit est extrait à l'acétate d'éthyle, séché sur Na₂SO₄ et les solvants sont évaporés.

L'huile obtenue est purifiée par chromatographie sur colonne de gel de silice avec comme éluant un mélange de CH₂Cl₂/MeOH 9/1.

| | |
|---|---|
| huile jaune | 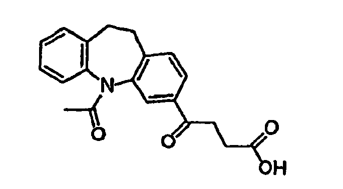 |
| MM (C₂₀H₁₉NO₄) : 337,38 g | |
| masse : 900 mg | |
| Rdt. 63% | |

RMN (¹H, 300 MHz, DMSO) : 12,17 (large s, 1H, COOH) ; 8,11 - 7,22 (m, 7H, 7CHarom.) ; 3,47 - 3,22 (m, 4H, -CH₂-CH₂-) 2,94 - 2,84 (m, 2H, -CH₂-) ; 2,59 (t, 2H, -CH₂-, J³= 6,2 Hz); 1,93 (s, 3H, -CH₃).

### 2) Procédé 2 : réaction de Friedel et Crafts avec le méthyl-4-chloro-4-oxo-butanoate

Exemple : synthèse du méthyl 4-(7-méthyl-imidazo[1,2-a]pyridin-3-yl)-4-oxobutanoate (dérivé céto-ester méthylique du composé N°21). Le composé 21 et son dérivé céto-ester ne sont pas selon l'invention.

On dissout le 7-méthyl imidazo [1,2-a]pyridine (0,50 g, 3,62 mmoles) dans du CS₂ (8 ml). On Refroidit la solution à 0°C et on additionne par petites portions le chlorure d'aluminium (1,40 g, 5,35 mmoles). On agite pendant 30 minutes puis on porte le milieu réactionnel à reflux. On ajoute le chlorure d'acide (1,50 g, 10 mmoles) et on agite à reflux pendant deux heures. On refroidit la solution que l'on verse sur de la glace. On ajuste le pH à 9 par ajout de NaOH 3N. On extrait ensuite à l'acétate d'éthyle puis on filtre sur terre d'infusoire. La phase d'acétate d'éthyle est séchée sur du sulfate de sodium puis évaporée à sec. Enfin, on triture à l'éther et l'on filtre le produit obtenu.

| | |
|---|---|
| poudre marron claire | 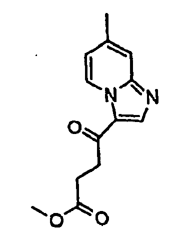 |
| MM (C₁₃H₁₄N₂O) : 246,26 g | |
| masse:100 mg | |
| Rdt. :12% | |

RMN (¹H, 300 MHz, CDCl₃) : 9,50 (d, 1H, CHarom., J³ = 6,7 Hz) ; 8,40 (s, 1H, CHimidaz.) ; 7,50 (s, 1H, CHarom.) ; 6,91 (d, 1H, CHarom., J³ = 6,7 Hz) ; 3,75 (s, 3H, - CH₃) ; 3,28 (t, 2H, -CH₂-, J³ = 6,7 Hz) ; 2,81 (t, 2H, -CH₂-, J³= 6,7 Hz) ; 2,50 (s, 3H, -CH₃).

### 3) Procédé 3 : Réaction de Friedel et Crafts avec l'anhydride glutamique

### Exemple : synthèse de l'acide 4-(4-méthoxybenzoyl)butyrique (non selon l'invention),

Dans un bicol sec de 250 ml sous argon à 0°C on place de l'anhydride glutarique (5 g, 87,64 mmoles) dans un mélange de CH₂Cl₂ (125 ml) et d'anisole (14,2 g, 1,5 éq., 0,13 mole). Du AlCl₃ (12,3 g, 1,05 éq., 92 mmoles) est ajouté par portions et le mélange agité à 0°C pendant 4 heures avant de le verser sur de la glace (100 g). Le précipité est collecté par filtration, lavé à l'eau froide et séché sous vide. On obtient 9 g de produit désiré.

| | |
|---|---|
| 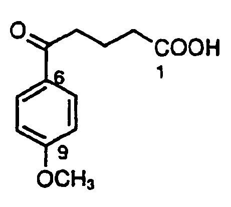 | FB : C₁₂H₁₄O₄ |
| | MM : 222,24 |
| Rendement : 50% | |
| Solide blanc, PF = 133°C | |

RMN ¹H (200 MHz, CDCl₃) : δ 2,08 (p, 2H, ³J = 7,2 Hz, H3) ; 2,50 (t, 2H, ³J = 7,2 Hz, H2) ; 3,04 (t, 2H, ³J = 7,2 Hz, H4) ; 3,88 (s, 3H, OCH₃) ; 7,47 (système AB, 4H arom., J_{AB} = 8,8 Hz, Δν = 203 Hz).

RMN ¹³C (50 MHz, CDCl₃) : δ 19,18 (C3); 33,04 (C2 ou C4) ; 36,94 (C2 ou C4); 55,46 (OCH₃) ; 113,72 (C8) ; 129,78 (C6) ; 130,30 (C7) ; 163,48 (C9) ; 178,80 (Cl) ; 197,99 (C5).

| Microanalyse : | C% | H% |
|---|---|---|
| théorique | 64,85 | 6,35 |
| trouvée | 64,71 | 6,20 |

SM (IE, 70 eV) m/e (intensité relative %) : 222 (M⁺, 78), 135 [(M-(CH₂)₃COOH)⁺, 100].

### 6) Procédé 4 : Réaction d'une méthyl-cétone avec l'acide glyoxalique

### Exemple : synthèse de l'acide (E)-4-(1H-indol-3-yl)-4-oxobut-2-ènoïque (non selon l'invention).

### On mélange 0,09 ml de cétone et 0,1 mole d'acide glyoxylique puis on porte ce dernier à 140°C pendant 20 heures. On surmonte ensuite le ballon d'un appareil de Hieckmann (déplacement de l'eau) et l'on chauffe à 110 °C pendant 10 heures et 145 °C pendant 3 heures. Le brut de réaction est repris à l'acétate d'éthyle et extrait au bicarbonate de potassium. On acidifie la phase aqueuse avec HCl concentré à 0°C et on filtre le précipité obtenu.

| | |
|---|---|
| Solide beige | 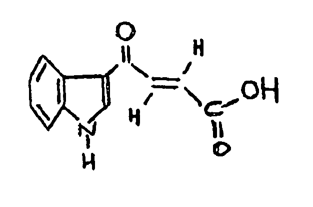 |
| MM (C₁₂H₉NO₃) : 215,21 g | |
| Rdt. : 62% | |
| T_{F} > 200°C | |

RMN (¹H 200 MHz" CDCl₃) : 8,10 - 8,30 (m, 2H, CH indol. +NH) ; 6,9 - 7,7 (m, 4H) ; 7,80 (d, 1H, CH_{A} = C, J³=16,0 Hz) ; 6,70 (d, 1H, CH_{B}= C, J³=16,0 Hz).

### 7) Procédé 5 : Réaction du diéthyl-succinate avec les arylesters

### Exemple : a) synthèse du diéthyl 2-(pyridin-3-ylcarbonyl)succinate (non selon l'invention)

L'éthyl nicotinate (10 ml, 73 mmoles) est dissout dans l'éther (50 ml). On ajoute le diéthyl succinate (24 ml, 144 mmoles), puis par petites spatules NaH (7,00 g, 60% dans l'huile, 175 mmoles). Le milieu réactionnel est agité à température ambiante sous argon pendant 2 jours. On le verse ensuite sur de la glace pilée.

La phase aqueuse est recueillie et traitée avec HCl 6N pour ramener le pH à 4,5. Le produit est extrait au chloroforme et séchée sur Na₂SO₄. Après évaporation des solvants, l'huile brute obtenue est purifiée par chromatographie sur colonne de gel de silice, avec comme éluant AcOEt/Hexane 1/2 puis 1/1 et 7/3.

| | |
|---|---|
| huile épaisse orange | 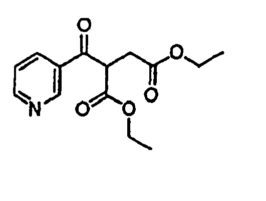 |
| MM (C₁₄H₁₇NO₅) : 279,29 g | |
| masse : 8,87 g | |
| Rdt. : 43% | |

RMN (¹H, 300 MHz, CDCl₃) : 9,25 (d, 1H, CHarom., J⁴= 2,0 Hz) ; 8,81 (dd, 1H, CHarom., J³ = 4,8 Hz et J⁴ = 2,0 Hz) ; 8,33 - 8,29 (m, 1H, CHarom.) ; 7,47 - 7,43 (m, 1H, CHarom.) ; 4,82 (m, 1H, -CH-) ; 4,18 - 4,09 (m, 4H, 2CH₂) ; 3,24 - 2,99 (m, 2H, CH₂-COO-); 1,28 - 1,13 (m, 6H, 2-CH₃).

### b) synthèse de l'acide 4-oxo-4-pyridin-3-ylbutanoïque (non selon l'invention)

Le céto-ester précédemment synthétisé (8,87 g, 31,76 mmoles) est dissout dans l'acide sulfurique 1N (80 ml). Le milieu réactionnel est agité à reflux pendant 3 heures puis refroidi. Le pH est ramené à 4,5 par addition goutte à goutte d'une solution saturée de NaHCO₃. Le précipité formé est filtré et séché.

| | |
|---|---|
| solide beige | 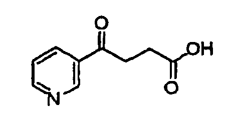 |
| MM (C₉H₉NO₃) : 179,18 g | |
| masse : 5,56 g | |
| Rdt. : 98% | |

RMN (¹H, 300 MHz, DMSO) : 9,17 (s, 1H, CHarom.) ; 8,80 (d, 1H, CHarom., J³ = 4,3 H ; 8,32 (d, 1H, CHarom., J³ = 7,6 Hz) ; 7,58 (dd, 1H, CHarom., J³ = 4,3 Hz et J³ = 7,6 Hz) ; 3,31 (t, 2H, -CH₂-, J³ = 6,2 Hz) ; 2,62 (t, 2H, -CH₂-, J³ = 6,2 Hz).

SM (IE, 70 eV) m/e (intensité relative %) : 208 (M⁺, 94), 152 [(M - CH₂CH₂CO)⁺, 72], 124 [(H-C-Ph-pCl)⁺, 57], 89 (100).

### B) Réduction des céto-acides ou céto-esters intermédiaires de formule générale II en alcools III correspondants

### 1) Réduction des céto-acides

### Exemple : synthèse de l'acide 4-hydroxy-4-(1-tosyl-1H-pyrrol-3-yl)butanoïque (composé N°4 n'est pas selon l'invention).

Le céto-acide (2,00 g, 62 mmoles) est dissout dans uns solution aqueuse 1M de KHCO₃ (20 ml). On ajoute par petites spatules KBH₄ (540 mg, 10 mmoles). Le milieu réactionnel est agité à température ambiante pendant 12 heures puis refroidi à 0°C. On ajoute goutte à goutte HCl concentré pour amener le pH à 1. On laisse ensuite agiter à froid pendant 1 heure puis on filtre le précipité formé que l'on laisse sécher à l'étuve.

| | |
|---|---|
| solide blanc | 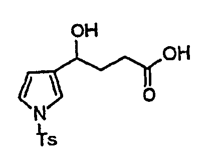 |
| MM (C₁₅H₁₇NO₅S) : 323,28 g | |
| masse : 1,80 g | |
| Rdt. : 89,5% | |

RMN (¹H, 300 MHz, DMSO) : 7,85 (d, 2H, 2CHarom., J³= 8,1 Hz) ; 7,44 (d, 2H, 2CHarom., J³ = 8,1 Hz) ; 7,26 (s, 1H, CHpyrrol.); 7,12 (s, 1H, CHpyrrol.); 6,32 (s, 1H, CHpyrrol.); 4,42 (t, 1H, CH-O-, J³ = 6,2 Hz) ; 2,39 (s, 3H, -CH₃) ; 2,20 (m, 2H, -CH₂-) 1,77 (m, 2H, -CH₂-).

### 2) Réduction des céto-esters de formule générale II par passage aux lactones de formule général VI

### Exemple : synthèse de la 5-(1H-indol-5-yl)dihydrofuran-2(3H) -one

Le céto-ester (300 mg, 1,10 mmoles) est dissout dans le MeOH sec (10 ml). Ajouter NaBH₄ (46 mg, 1,22 mmoles). Le milieu réactionnel est agité à température ambiante pendant 24 heures, en ajoutant toutes les 6 heures une pointe de spatule de NaBH₄. On évapore le méthanol et on reprend le résidu dans un mélange eau/AcOEt. La phase organique est receuillie, séchée sur Na₂SO₄ et évaporée.

Le brut est purifié par chromatographie sur colonne de gel de silice avec comme éluant CH₂Cl₂/AcOEt 9/1.

| | |
|---|---|
| poudre blanche | 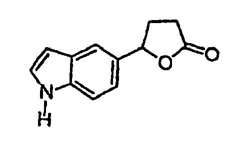 |
| MM (C₁₂H₁₁NO₂) : 201,23 g | |
| masse : 110 mg | |
| Rdt. : 47% | |

RMN (¹H, 300MHz, CDCl₃) : 8,58 (large s, 1H, NH) ; 7,61 (s, 1H, CHarom.) ; 7,38 (d, 1H, CHarom., J³ = 9,3 Hz) ; 7,23 (m, 1H, CHindol.) ; 7,13 (d, 1H, CHarom., J³ = 9,3 Hz) ; 6,55 - 6,54 (m, 1H, CHindol.) ; 5,58 (m, 1H, CHlacton.) ; 2,76 - 2,57 (m, 3H, CH₂-CH) ; 2,38 - 2,22 (m, 1H, CH).

Les alcools-acides peuvent être lactonisés par hydrolyse à chaud (60°C, HCl conc. dans THF) pendant une nuit.

### C) Accès aux sels de sodium (produits finaux)

Les alcools-acides III obtenus par réduction suivie de l'hydrolyse ou ouverture des lactones IV sont salifiés par une solution alcaline, par exemple par une solution de soude 1M dans l'eau (0,9 éq.). La phase aqueuse est lavée à l'acétate d'éthyle, puis lyophilisée ou évaporée suivie d'une trituration à l'éther.

Cette réaction de salification est bien connue de l'homme du métier et ne nécessite pas d'être développée plus en détail ici. Bien entendu, l'homme du métier pourra choisir à la place de l'hydroxyde de sodium précité toute autre base adaptée pour obtenir les sels correspondant souhaités. A titre d'exemples de bases adaptées on peut citer, de manière non-limitative, les bases minérales telles que LiOH, Ca(OH)₂, etc., ainsi que les bases organiques habituellement utilisées en chimie organique de synthèse, en particulier celles utilisées pour la synthèse de compositions à destinée pharmaceutique.

### D) Cas particuliers :

### 1) Formation des amides de formule générale V (cf. figure 2)

Comme indiqué sur le schéma de la figure 2, l'ouverture des lactones réduites IV peut se faire par ajout d'une amine primaire ou secondaire ou par ajout d'un acide hydroxamique, pour aboutir aux amides ou aux composés hydroxamino-substitués correspondants V. Ci-après, on décrira plus en détail trois modes de réalisations donnés à titre d'exemples illustratifs relatifs à cette opération. Les composés des exemples 1-3 ne sont pas selon l'invention.

### Exemple 1: synthèse d'un amide primaire, le 4-(1-benzothiophène-2-yl)-4-hydroxybutanamide (dérivé amide du composé N° 18)

On dissout la lactone (103 mg, 0,47 mmole) dans le THF (5 ml) et on ajoute NH₄OH (2 ml, 25% dans de l'eau). On chauffe à 50°C pendant 1 h 30 puis on laisse refroidir et évaporer le THF. On reprend le résidu dans AcOEt et on sèche sur Na₂SO₄. Les solvants sont évaporés et l'huile brute obtenue est purifiée par chromatographie sur colonne de gel de silice, avec comme éluant CH₂Cl₂/MeOH 9/1.

| | |
|---|---|
| Huile épaisse | 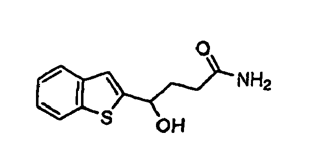 |
| MM (C₁₂H₁₃NO₂S) : 235,31 g | |
| masse: 61 mg | |
| Rdt. : 55% | |

RMN (1H, 300 MHz, CDCl₃) : 7,89 - 7,85 (m, 2H, 2CHarom.) ; 7,44 (s, 1H, CHthienyl) ; 7,41 - 7,33 (m, 2H, 2CHarom.) 5,59 et 5,45 (2s, 2H, -NH₂) ; 5,24 (m, 1H, CH-O) ; 3,50 (s, 1H, -OH) ; 2,50 - 2,21 (m, 4H, -CH₂-CH₂-).

### Exemple 2 : synthèse d'un amide secondaire, le 4-(1-benzothiophène-2-yl)-N-benzyl-4-hydroxybutanamide

La lactone (0,102 g, 0,47 mmole) est dissoute dans l'éther (5 ml). On ajoute la benzylamine (51 µl ; 0,47 mmole) et on laisse agiter le milieu réactionnel pendant 48 heures. On évapore l'éther et on purifie le brut par chromatographie sur colonne de gel de silice, avec comme éluant CH₂Cl₂/MeOH 95/5.

| | |
|---|---|
| huile translucide très épaisse | 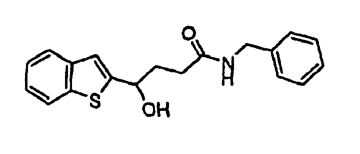 |
| MM (C₁₉H₂₀NO₂S) : 326,45 g | |
| masse : 107 mg | |
| Rdt. : 70% | |

RMN (¹H, 300 MHz, CDCl₃) : 7,84 (m, 2H, 2CHarom.) ; 7,43 - 7,28 (m, 8H, 8CHarom.) ; 5,89 (large s, 1H, -OH) ; 5,24 (m, 1H, CH-O) ; 4,46 (d, 2H, CH2, J³ = 5,0 Hz) ; 3,82 (d, 1H, -NH, J³ = 5,0 Hz) ; 2,47 - 2,19 (m, 4H, -CH₂-CH₂-).

### Exemple 3 : traitement avec l'acide hydroxamique pour l'obtention du N,4-dihydroxy-4-phénylbutanamide (composé N°34)

Le sodium métallique (100 mg, 4,34 mmoles) est dissout dans le méthanol (4 ml). On ajoute l'hydroxylamine (0,30 g, 4,32 mmoles) puis la lactone (0,50 g, 3,09 mmoles) dissoute dans le méthanol (5 ml). On agite le milieu réactionnel à température ambiante pendant 12 heures. Le méthanol est évaporé et le résidu repris dans l'acétate d'éthyle. Les insolubles sont filtrés et le filtrat séché sur Na₂SO₄. On évapore les solvants et l'huile obtenue est triturée à l'éther.

| | |
|---|---|
| solide blanc très hygroscopique | 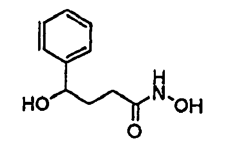 |
| MM (C₁₀H₁₃NO₃) : 195,22 g | |
| masse : 220 mg | |
| Rdt. : 37% | |

RMN (¹H, 300 MHz, DMSO) : 7,41 - 7,21 (m, 5H, 5CHarom.) ; 4,55 (m, 1H, -CH-O) ; 2,04 - 1,73 (2m, 4H, -CH₂-CH₂-).

### 2) Synthèses de composés de type VIII, IX et X

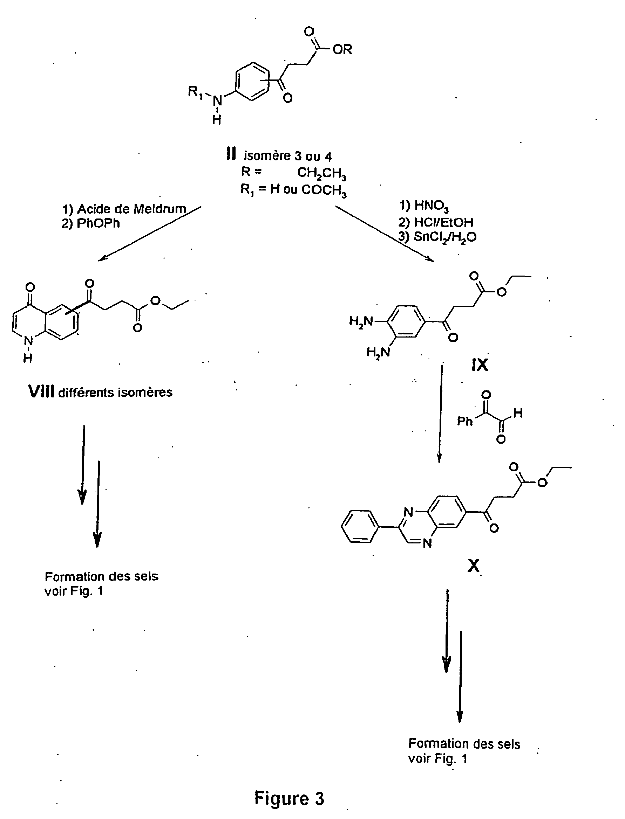

Comme montré sur le schéma de la figure 3, la présence, en position 3 ou 4 d'une amine sur le β-benzoylpropionate d'éthyle (composé de formule générale II avec Ar = PhNH₂, n = 0, X = (CH₂)₂ et R = Et) a permis la synthèse des dihydroquinolones (composés VIII) et phénylquinoxalines (composés X).

En effet, la condensation de l'amine avec l'acide de Meldrum conduit intermédiairement à l'amine méthyline-dioxane-dione (composé IX) qui se cyclise thermiquement (cf. schéma de gauche de la figure 3).

La réaction avec le phénylglyoxal conduit à la phénylquinoxaline de formule X attendue.

Les céto-esters obtenus sont ensuite réduits en lactones et salifiés comme décrit dans le schéma de la figure 1.

Les exemples indicatifs suivants décrivent plus en détails les conditions opératoires utilisées pour ces types de réactions.

### a) Synthèses de composés de type VIII

### Exemple 1 : synthèse du éthyl 4-(4-{[2,2-diméthyl-4,6-dioxo-1,3-dioxan-5-ylidène)méthyl]amino}phényl)-4-oxobutanoate

Le mélange, acide de Meldrum (0,70 g, 0,46 mmole) et méthyl orthoformiate (15 ml) est chauffé à reflux (140°C) pendant 2 heures. L'amine (1,00 g, 4,52 mmoles) est ajoutée en une seule fois. Le milieu réactionnel est agité à reflux pendant 12 heures. Après refroidissement, le solvant est évaporé et le résidu est purifié par recristallisation dans le méthanol.

| | |
|---|---|
| poudre or-orangée | 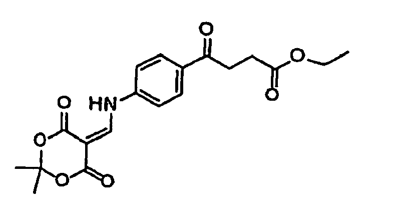 |
| MM (C₁₉H₂₁NO₇): 375,39 g | |
| masse : 1,26 g | |
| Rdt. : 75% | |
| Tf: 154°C | |

RMN (¹H, 300 MHz, CDCl₃) : 11,32 (large d, 1H, -NH, J³ = 14,0 Hz) ; 8,70 (d, 1H, CH, J³ = 14,0 Hz) ; 8,07 (m, 2H, 2CHarom.) ; 7,34 (m, 2H, 2CHarom.) ; 4,15 (q, 2H, -CH₂-, J³ = 7,2 Hz) ; 3,29 (t, 2H, -CH₂-, J³ = 6,5 Hz) ; 2,77 (t, 2H, -CH₂-, J³ = 6,5 Hz) ; 1,76 (s, 6H, 2-CH3) ; 1,27 (t, 3H, -CH₃, J³= 7,2 Hz).

### Exemple 2 : synthèse du éthyl 4-oxo-4-(4-oxo-1,4-dihydroquinolin-6-yl)-butanoate

Le diphényl éther (10 ml) est chauffé à 240°C (léger reflux), le dérivé de Meldrum (1,00 g, 2,66 mmoles) est ajouté en une seule fois. Le milieu réactionnel est ensuite agité à 240°C pendant 10 minutes, puis versé très doucement sur de l'éther de pétrole (150 ml). Le précipité formé est filtré et purifié par chromatographie sur colonne de gel de silice (éluant : CH₂Cl₂/MeOH 9/1).

| | |
|---|---|
| solide beige | 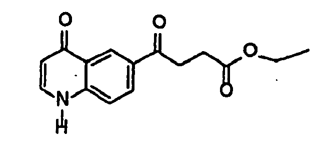 |
| MM (C₁₅H₁₅NO₄) : 273,33 g | |
| masse: 0,52 g | |
| Rdt. : 71% | |

RMN (¹H, 200 MHz, CDCl₃ + MeOD) : 8,90 (d, 1H, CHarom., J⁴ = 2,0 Hz) ; 8,13 (dd, 1H, CHarom., J³ = 8,8 Hz, J⁴ = 2,0 Hz) ; 7,62 (d, 1 H, CHvinyl., J³ = 7,3 Hz) ; 7,37 (d, 1H, CHarom., J³ = 8,8 Hz) ; 6,30 (d, 1H, CHvinyl., J³ = 7,3 Hz) ; 4,16 (q, 2H, -CH₂-, J³ = 7,1 Hz) ; 3,41 (t, 2H, -CH₂-, J³ = 6,6 Hz) ; 2,77 (t, 2H, -CH₂-, J³ = 6,6 Hz) ; 1,26 (t, 3H, -CH₃, J³ = 7,1 Hz).

### b) Synthèses de composés de type X

### Exemple 1 : l'acide 4-[4-(acétylamino)-3-nitrophényl]-4-oxobutanoïque

On refroidit HNO₃ fumant (12 ml) à 0°C puis on ajoute rapidement le dérivé à nitrer (4,00 g, 17,0 mmoles). On laisse agiter à 0°C pendant 10 minutes. Ensuite, on verse le milieu sur de la glace et on filtre le précipité formé.

| | |
|---|---|
| poudre jaune | 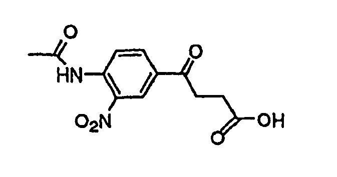 |
| MM (C₁₂H₁₂N₂O₆): 280,23 g | |
| masse : 2,97 g | |
| Rdt. : 62% | |
| Tf : 145°C | |

RMN (¹H, 200 MHz, DMSO) : 10,6 (s, 1H, NH) ; 8,44 (d, 1H, CHarom., J⁴ = 2,0 Hz) ; 8,29 (dd, 1H, CHarom., J³ = 8,5 Hz, J⁴ = 2,0 Hz) ; 7,86 (d, 1H, CHarom., J³ = 8,5 Hz) ; 3,30 (t, 2H, -CH₂-, J³ = 6,2 Hz) ; 2,61 (t, 2H, -CH₂-, J³ = 6,2 Hz).

### Exemple 2 : l'éthyl 4-(4-amino-3-nitrophényl)-4-oxobutanoate

On mélange le dérivé N-acétylé (2,00 g, 7,51 mmoles) avec HCl concentré (10 ml) et on chauffe à reflux pendant 15 minutes. Puis, on ajoute EtOH (50 ml) et on agite à reflux pendant 12 heures. On laisse ensuite refroidir et évaporer au maximum les solvants. On extrait le résidu à l'acétate d'éthyle et on ramène le pH à 8-9 par addition de KHCO₃. On sèche la phase organique et on évapore les solvants. Enfin, on purifie le brut par chromatographie sur colonne de gel de silice (éluant : AcOEt/Hexane 1/3).

| | |
|---|---|
| huile orangée | 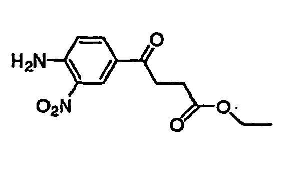 |
| MM (C₁₂H₁₄N₂O₅) : 266,25 g | |
| masse : 1,36 g | |
| Rdt. : 68% | |

RMN (¹H, 300 MHz, CDCl₃) : 8,81 (d, 1H, CHarom., J⁴ = 2,0 Hz) ; 8,02 (dd, 1H, CHarom., J³ = 8,8 Hz, J⁴ = 2,0 Hz) ; 6,86 (d, 1H, CHarom., J³ = 8,8 Hz) ; 4,17 (q, 2H, -CH2-, J³ = 7,1 Hz) ; 3,26 (t, 2H, -CH₂-, J³ = 6,5 Hz) ; 2,77 (t, 2H, -CH₂-, J³ = 6,5 Hz) ; 1,27 (t, 3H, -CH₃, J³ = 7,1 Hz).

### Exemple 3 : l'éthyl 4-(3,4-diaminophényl)-4-oxobutanoate

On dissout le dérivé nitré (1,31 g, 4,9 mmoles) dans l'éthanol à 95% (30 ml). On rajoute SnCl₂, 2H₂O (4,4 g, 19,6 mmoles) et on chauffe à reflux pendant 2 heures. On évapore les solvants et on extrait le résidu à l'acétate d'éthyle ; puis on ramène le pH à 7 par ajout de KHCO₃. On filtre le précipité de sels d'étain formé, on sèche la phase organique sur Na₂SO₄ et on évapore à sec. Enfin, la purification du brut s'effectue par chromatographie sur colonne de gel de silice (éluant : AcOEt/Hexane 1/1).

| | |
|---|---|
| Huile épaisse | 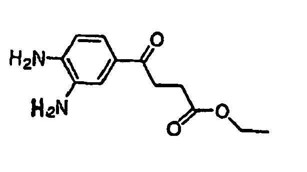 |
| MM (C₁₂H₁₆N₂O₃): 236,01 g | |
| masse : 700 mg | |
| Rdt. : 60% | |

RMN (¹H, 300MHz, CDCl₃) : 7,46 - 7,38 (m, 2H, 2CHarom.) ; 6,68 (d, 1H, CHarom., J³ = 8,1 Hz) ; 4,16 (q, 2H, -OCH₂-) ; 3,89 (large s, 2H, -NH₂) ; 3,37 (large s, 2H, -NH₂) ; 3,22 (t, 2H, -CH₂-, J³ = 6,8 Hz) ; 2,72 (t, 2H, -CH₂-, J³ = 6,8 Hz) ; 1,27 (t, 3H, -CH₃).

### Exemple 4 : l'éthyl-4-oxo-4-(2-phénylquinoxalin-6yl)butanoate

Dans un ballon, on dissout le diamino-ester dans EtOH (10 ml). On ajoute le phényl glyoxal, hydrate (130 mg, 0,85 mmole) et on chauffe à reflux pendant 5 heures. On laisse refroidir et on filtre le précipite formé.

| | |
|---|---|
| poudre jaune | 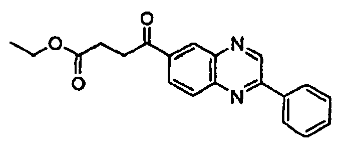 |
| MM (C₂₀H₁₈N₂O₃) : 334,37 g | |
| masse : 233 mg | |
| Rdt. : 82% | |

RMN (¹H, 200 MHz, CDCl₃) : 9,43 (s, 1H, CHquinox.) ; 8,74 (d, 1H, CHarom., J⁴ = 1,9 Hz) ; 8,36 (dd, 1H, CHarom., J³ = 8,9 Hz, J⁴ = 1,9 Hz) ; 8,26 - 8,19 (m, 4H, 4CHarom.) ; 7,60 - 7,58 (m, 3H, 3CHarom.) ; 4,19 (q, 2H, -CH₂-, J³ = 7,1 Hz) ; 3,50 (t, 2H, -CH₂-, J³ = 6,6 Hz) ; 2,86 (t, 2H, -CH₂-, J³ = 6,6 Hz) ; 1,30 (t, 3H, -CH₃, J³ = 7,1 Hz).

### E) Synthèses de composés énantiomériquement purs

La présente invention décrit également la synthèse énantiosélective des sels de sodium des acides (R) et (S) de l'acide γ-benzyl-γ-hydroxybutanoïque. Ces synthèses mettent en jeu l'acide L ou D glutamique comme illustré dans le schéma de la figure 4. Pour cela, il a été préparé les chlorures d'acides (R) et (S) optiquement purs d'après des procédés décrits dans la littérature (réf. 1 : M. Larchevèque et al., Bull. Soc. Chim. Fr. 1987, 116-122 ; G. Eguchi et al., Bull. Chim. Soc. Jpn., 1974, 47, 1704-1708).

Ces composés ont ensuite été traités par du tétraphényl étain en présence d'un catalyseur dans HMPT (réf. 2 : J.W. Labadie et al., J.A.C.S., 1983, 105, 6129). On obtient alors les γ-benzoyl-γ-butyrolactones (R) et (S) qui sont réduites par hydrogénation catalytique (procédé 4) pour donner les deux énantiomères optiquement purs (composés VI' (R) et VI" (S)). Ceux-ci sont salifiés comme précédemment décrit pour conduire aux sels de sodium (R) et (S) correspondants.

### F) Caractérisation des composés obtenus

Le tableau suivant donne les principales caractéristiques physico-chimiques de composés spécifiques appartenant aux familles de formules générales I, I' ou I" tels que synthétisés en pratique et qui sont explicitement revendiqués dans la présente invention. Bien entendu, les composés acides correspondants de formule générale I ou I' où W est COOH qui ont permis l'obtention des sels sodiques sont également considérés comme faisant partie de ce tableau et des composés explicitement revendiqués.

**Tableau 2 : Principales caractéristiques physico-chimiques des composés obtenus**

| N° du composé | Nom | N° du Procédé | formule brute | Masse molaire g/mol | RMN "¹H", 200 ou 300 MHz, solvant ou microanalyses |
|---|---|---|---|---|---|
| 2 | Sel de sodium de l'acide 4-(3,4 dichlorophényl)-4-hydroxybutanoïque | 1 | C₁₀H₉Cl₂O₃Na | 271,08 | ¹H, 200 MHz, D₂O : 7,35 - 7,30 (m, 2H, 2CHarom.) ; 7,11 - 7,06 (m, 1H, CHarom.) ; 4,50 (t, 1H, CH-O-, J³ = 6,6 Hz) ; 2,08 - 2,00 (m, 2H, -CH₂-) ; 1,88 - 1,75 (m, 2H, -CH₂-) |
| 3 | Sel de sodium de l'acide 4-(3,4-diméthoxylphényl)-4-hydroxybutanoïque | 1 | C₁₂H₁₅O₅Na | 262,23 | ¹H, 200 MHz, D₂O : 6,97 (m, 3H, CHarom.) ; 4,59 (t, 1H, CH-O-, J³ = 8,3 Hz) ; 3,82 (s, 3H, -O-CH₃-) ; 3,80 (s, 3H, -O-CH₃-) ; 2,16 - 1,89 (m, 4H, -CH₂-CH₂-) |
| 10 | Sel de sodium de l'acide 4-(2,3-dihydro-1-benzofuran-5-yl)-4-hydroxybutanoïque | 1 | C₁₂H₁₃O₄Na | 244,22 | ¹H, 300 MHz, D₂O : 7,17 (s, 1H, CHarom.) ; 7,01 (d, 1H, CHarom., J³= 8,1 Hz) ; 6,69 (d, 1H, CHarom., J³ = 8,1 Hz) ; 4,47 (m, 3 H, -CH₂ et CH-O) ; 3,09 (t, 2H, -CH₂-, J³= 8_{,}7 Hz) ; 2,08 - 1,78 (m, 4H, -CH₂-CH₂) |
| 12 | Sel de sodium de l'acide 4-(1-acétyl-2,3-dihydro-1*H*-indol-5-yl)-4-hydroxybutanoïque | 1 | C₁₄H₁₆NO₄Na | 285,28 | ¹H, 200 MHz, D₂O) : 7,82 (d, 1H, CHarom., J³ = 8,6 Hz) ; 7,16 (s, 1H, CHarom.) ; 7,08 (d, 1H, CHarom., J³ = 8,6 Hz) ; 4,52 (t, 1H, -CH-O, J³ = 6,4 Hz) ; 3,93 (t, 2H, -CH₂, J³ = 8,2 Hz) ; 3,02 (t, 2H, -CH₂, J³ = 8,2 Hz); 2,07 (s, 3H, -CH₃) ; 2,04 - 1,88 (2m, 4H, -CH₂-CH₂-) |
| 15 | Sel de sodium de l'acide 4-(2,3-dihydro-1*H* inden-5-yl)-4-hydroxy butanoïque | 1 | C₁₃H₁₅O₃Na | 242,25 | ¹H, 300 MHz, D₂O : 7,17 (s, 1H, CHarom.) ; 7,15 (s, 1H, CHarom.) ; 7,15 (d, 1H, CHarom., J³ = 7,6 Hz) ; 7,05 (d, 1H, CHarom., J³ = 7,6 Hz) ; 4,54 (t, 1H, CH-O, J³ = 6,5 Hz) ; 2,80 - 2,73 (m, 4H, 2-CH₂) ; 2,14 - 1,82 (m, 3H, 3-CH₂) |
| 16 | Sel de sodium de l'acide 4-[2-(éthoxycarbonyl)-1*H*-indol-5-yl]-4-hydroxybutanoïque | 1 | C₁₅H₁₆NO₅Na | 313,28 | ¹H, 200 MHz, D₂O : 7,56 (s, 1H, CHarom.) ; 7,32 - 7,36 (m, 2H, 2CHarom.) ; 7,05 (s, 1H, CHindol.) ; 4,71 (m, 1H, -CH-O) ; 4,20 (q, 2H, -CH₂-) ; 2,20 - 2,10 (m, 4H, -CH₂-CH₂-) ; 1,3 (t, 3H, -CH₃-) |
| 17 | Sel de sodium de l'acide 4-hydroxy-4-(1*H-*indol-5-yl)-butanoïque | 1 | C₁₂H₁₂NO₃Na | 241,22 | ¹H, 200 MHz, D₂O : 7,55 (s, 1H, CHarom.) ; 7,43 (d, 1H, CHarom., J³ = 8,4 Hz) ; 7,30 (d, 1H, CHindol., J³ = 2,9 Hz) ; 7,13 (d, 1H, CHarom., J³ = 8,4 Hz) ; 6,48 (d, 1H, CHindol., J³ = 2,9 Hz) ; 4,70 (en dessous du pic de l'eau, 1H, CH-O) ; 2,17 - 1,91 (m, 4H, -CH₂-CH₂-) |
| 19 | Sel de sodium de l'acide 4-hydroxy-4-(4-oxo-1,4-dihydroquinolin-7-yl)-butanoïque | cas particulier | C₁₃H₁₂NO₄Na | 269,23 | ¹H, 300 MHz, D₂O : 8,08 (d, 1H, CHarom., J³ = 8,1 Hz); 8,00 (d, 1H, CHarom., J³ = 7,2 Hz); 7,54 (s, 1H, CHarom.) ; 7,41 (d, 1H, CHarom., J³ = 8,1 Hz) ; 6,37 (d, 1 H, CHarom., J³ = 7,2 Hz) ; 4,80 (t, -CH-O, J³ = 6,4 Hz ) ; 2,15 (t, 2H, -CH₂, J³ = 7,2 Hz) ; 1,99 (t, 2H, -CH₂, J³ = 7,2 Hz) |
| 20 | Sel de sodium de l'acide 4-hydroxy-4-(4-oxo-1,4-dihydroquinolin-6-yl)-butanoïque | cas particulier | C₁₃H₁₂NO₄Na | 269,23 | ¹H, 300 MHz, D₂O : 7,93 (d, 1H, CHarom., J⁴ = 1,9 Hz) ; 7,86 (d, 1H, CHvinyl., J³ = 7,2 Hz) ; 7,61 (dd, 1H, CHarom., J³ = 8,8 Hz, J⁴ = 1,9 Hz); 7,45 (d, 1H, CHarom., J³ = 8,8 Hz) ; 6,27 (d, 1H, CHvinyl., J³ = 7,2 Hz) ; 4,75 (t, 1H, -CH-O, J³ = 3,7 Hz) ; 2,15 (t, 2H, -CH₂, J³ = 7,2 Hz) ; 2,17 - 1,94 (2m, 4H, -CH₂-CH₂-) |
| 22 | Sel de sodium de l'acide 4-hydroxy-4-(2-oxo-2,3-dihydro-1*H-*indol-5-yl)-butanoïque | 1 | C₁₂H₁₂NO₄Na | 257,28 | ¹H, 200 MHz, D₂O: 12,31 (s, 1H, NH) ; 7,16 (s, 1H, CHarom.) ; 7,08 (d, 1H, CHarom., J³ = 8,8 Hz) ; 6,74 (d, 1H, CHarom., J³ = 8,8 Hz) ; 4,47 (s, 1H, CHO) ; 3,44 (s, 2H, CH₂) ; 2,21 et 1,79 (2s, 4H, 2CH₂) |
| 23 | Sel de sodium de l'acide 4-(2-[(diméthylamino)carbon yl]-1*H*-indol-5-yl)-4-hydroxybutanoïque | 1 | C₁₅H₁₇N₂O₄Na | 312,30 | ¹H, 300 MHz, D₂O): 7,60 (s, 1H, CHarom.) 7,28 - ,7,42 (m, 2H, 2CHarom.) ; 7,05 (s, 1H, CHindol.) ; 3,35 (m, 4H, -CH₂-CH₂-) ; 2,50 (s, 6H, 2-CH₃) |
| 25 | Sel de sodium de l'acide 4-dibenzo[b,d]thiophène-2-yl-4-hydroxybutanoïque | 1 | C₁₆H₁₃O₃SNa | 308,34 | ¹H, 200 MHz, D₂O : 8,10 - 8,00 (m, 2H, 2CHarom.) ; 7,98 - 7,75 (m, 2H, 2CHarom.); 7,40 - 7,30 (m, 3H, 3CHarom.) ; 4,74 (m, 1H, -CH-O) ; 2,15 - 2,02 (m, 4H, -CH₂-CH₂-) |
| 26 | Sel de sodium de l'acide 4-dibenzo[b,d]furan-2-yl-4-hydroxybutanoïque | 1 | C₁₆H₁₃O₄Na | 292,27 | ¹H, 300 MHz, DMSO : 8,04 (m, 2H, 2CHarom.) ; 7,70 - 7,35 (m, 5H, 5CHarom.); 4,78 (m, 1H, -CH-O) ; 2,13 (2H, -CH₂); 1,84 (m, 2H, -CH₂-) |
| 27 | Sel de sodium de l'acide 4-(9-acétyl-9*H-*carbazol-3-yl-4-hydroxybutanoïque | 1 | C₁₈H₁₆O₄NNa | 333,33 | ¹H, 300 MHz, D₂O : 7,57 - 7,01 (m, 7H, 7CHarom.) ; 4,64 (m, 1H, -CH-O) ; 2,30 (s, 3H, -CH₃) ; 2,20 - 1,82 (2m, 4H, -CH₂-CH₂-) |
| 28 | Sel de sodium de l'acide 4-hydroxy-4-(phénoxathiin-2 ou 3-yl)butanoïque isomère A | 1 | C₁₆H₁₃O₄SNa | 324,34 | ¹H, 200 MHz, D₂O : 7,06 - 6,83 (m, 7H, 7CHarom.) ; 4,43 (m, 1H, -CH-O) ; 2,01 - 1,77 (2m, 4H, -CH₂-CH₂-) |
| 29 | Sel de sodium de l'acide 4-hydroxy-4-(phénoxathiin-2 ou 3-yl)butanoïque isomère B | 1 | C₁₆H₁₃O₄SNa | 324,34 | ¹H, 300 MHz, D₂O : 7,77 - 7,63 (m, 4H, 4CHarom.) ; 7,38-7,32 (m, 3H, 3CHarom.) ; 4,62 (m, 1H, -CHO); 2,23-1,96 (2m, 4H, -CH₂CH₂-) |
| 30 | Sel de sodium de l'acide 4-(5-acétyl-10,11-dihydro-5*H* dibenzo[b,f]azépin-3-yl)-4-hydroxybutanoïque | | C₂₀H₂₀NO₄Na | 361,38 | ¹H, 200 MHz, D₂O : 7,32 - 7,16 (m, 7H, 7CHarom.) ; 7,51 (m, 1H, -CH-O-) ; 3,27 - 3,07 (m, 2H, -CH₂-) ; 2,78-2,64 (m, 2H, -CH2-) ; 1,97 - 1,76 (m, 7H, -CH₂-CH₂- et -CH₃) |
| 31 | Sel de sodium de l'acide 4-hydroxy-4-(9*H*-xanthen-2-yl)butanoïque | 1 | C₁₇H₁₅O₄Na | 306,30 | ¹H, 200 MHz, D₂O : 7,16 - 6,91 (m, 7H, 7CHarom.) ; 4,58 (m, 1H, -CH-O) ; 3,86 (s, 2H, -CH₂) ; 2,11 - 1,97 (2m, 4H, -CH₂-CH₂) |
| 32 | Sel de sodium de l'acide 4-(9*H*-fluoren-3-yl)hydroxybutanoïque | 1 | C₁₇H₁₅O₃Na | 290,30 | ¹H, 300 MHz, D₂O : 7,65 (m, 2H, 2CHarom.) ; 7,45 (m, 2H, 2CHarom.) ; 7,29 - 7,23 (m, 3H, 3CHarom.) ; 4,62 (m, 1H, -CH-O) ; 3,37 (s, 2H, -CH2-) ; 2,17-1,91 (2m, 4H, -CH2-CH2-) |
| 33 | Sels de sodium des acides 4-hydroxy-4-(4-oxo-4,5-dihydro-3*H*-pyridazo(4,5-b)indol-8 et 6-yl)butanoïque mélange isomère 8/6 | 1 | C₁₄H₁₂N₃O₃Na | 293,25 | ¹H, 300 MHz, D₂O : 8,06 (s, 1H, CHarom. des 2 isomères) ; 7,50 (s, 1H, CHarom. des 2 isomères) ; 7,39 (d, 1H, CHarom. iso. 6, J³ = 7,6 Hz) ; 7,28 2d, 1H, CHarom. iso. 8 CHarom. iso., 6, J³ = 8,7 Hz) ; 7,16 (t, 1H, CHarom. iso. 8, J³ = 8,7 Hz) ; 7,06 (t, 1H, CHarom. iso. 6, J³ = 7,6 Hz) ; 4,90 (m, 1H, CH-O- iso. 6) ; 4,66 (m, 1H, CH-O- iso. 8) ; 2,29 - 1,95 (2m, 4H, -CH₂-CH₂- des 2 isomères) |
| 40 | Sel de sodium de l'acide 4-(3,3-diméthyl-2-oxo-1,2,3,4-tétrahydroquinolin-6-yl)-4-hydroxybutanoïque | | C₁₅H₁₆NO₄Na | 297,26 | ¹H, 200 MHz, D₂O : 7,30 (s, 1H, CHarom.) ; 7,13 (d, 1H, CHarom., J³ = 7,9 Hz) ; 6,84 (d, 1H, CHarom., J³ = 7,9 Hz) ; 6,55 (m, 1H, CH=C) ; 5,95 (d, 1H, CH=C, J³ = 10,8 Hz) ; 5,24 (d, 1H, CH-O, J³ = 3,3 Hz) ; 2,35 (s, 2H, CH₂) ; 1,17 (s, 6H, 2CH₃) |

### G) Tests d'activité thérapeutique

### 1) Tests in vitro

Une quarantaine de composés selon l'invention a été synthétisée et testée dans des expériences de liaison sur des cerveaux de rats en utilisant le ³H-GHB comme radio-ligand et selon le protocole expérimental décrit ci-après.

Pour les études de déplacement, le ligand de référence utilisé est le GHB tritié (100 Ci/mmol, CEA, Saclay). Les récepteurs étudiés proviennent de membranes de cerveaux de rats Wistar élevés dans le laboratoire. Les animaux sont rapidement tués par décapitation et les cerveaux sont prélevés en excluant le cervelet et le tronc cérébral.

Les cerveaux sont ensuite homogénéisés dans 10 volumes (poids/volume) de sucrose 0,32 M contenant 5 mM d'EDTA (pH 6,0). Après une première centrifugation à 800 g destinée à éliminer les débris cellulaires et les noyaux, le surnageant est centrifugé à 16 000 g de façon à obtenir le culot P₂ (synaptosomes + mitochondries).

Ce culot est ensuite homogénéisé dans un appareil adapté, par exemple du type connu sous la dénomination « polytron », dans 70 volumes d'eau distillée à 0°C contenant 5mM d'EDTA. Après recentrifugation à 20 000 g (4°C, 20 mn), le culot obtenu est lavé avec le même milieu supplémenté avec 0,5% de CHAPS (3-[3-cholamidopropyl-diméthylammonio]-1-propane sulfonate). Après une nouvelle centrifugation à 20 000 g, les membranes obtenues sont resuspendues dans un tampon phosphate de potassium à pH = 6,0. Après recentrifugation, le culot est stockée à - 80°C pour une utilisation le jour suivant.

L'incubation des membranes avec le GHB radioactif et les divers composés à des concentrations variables (comprises entre 10⁻⁹ M et 10⁻⁴ M) s'effectue dans un tampon phosphate de potassium également à pH = 6,0 pendant une durée de 30 minutes dans de la glace.

Au terme de cette incubation, la séparation du ³H-GHB lié du ³H-GHB libre s'effectue par filtration sous aspiration avec des filtres en fibre de verre (Whatmann GF/B). Les membranes retenues par le filtre sont rapidement lavées sous aspiration par trois fois 1 ml de tampon d'incubation maintenu à 0°C.

Les filtres sont ensuite « comptés » par scintillation liquide dans un compteur, en présence d'un liquide de scintillation. Les résultats sont exprimés en pourcentage de la fixation réversible totale déterminée en présence d'un excès de GHB non radioactif (500 µM, 60-80% de la fixation totale). Les analyses statistiques des CI₅₀ (concentration d'analogues synthétiques susceptibles de déplacer 50% de la fixation réversible du ³H-GHB) sont résumées dans le tableau ci-après. Plus le CI₅₀ est faible, plus l'affinité du ligand pour le récepteur est forte. Les analyses statistiques des courbes de déplacement sont réalisées à l'aide du logiciel GraphPad Prism (San Diego, CA).

Les résultats obtenus sont regroupés dans le tableau suivant :

**Tableau 3 : Résultats in vitro**

| | | | | | | | |
|---|---|---|---|---|---|---|---|
| N° du composé | CI₅₀ µM | | N° du composé | CI₅₀ µM | | N° du composé | CI₅₀ µM |
| GHB | 5,60 | | 17 | 1,40 | | 28 | 0,10 |
| 2 | 0,30 | | 19 | 1,10 | | 29 | 0,90 |
| 3 | 0,30 | | 20 | 24,7 | | 30 | 0,50 |
| | | | 22 | 1,20 | | 31 | 0,10 |
| 10 | 0,60 | | 23 | 0,20 | | 32 | 0,30 |
| 12 | 0,60 | | 25 | 0,1 | | 33 | 0,20 |
| 15 | 0,70 | | 26 | 0,30 | | 40 | 1,10 |
| 16 | 0,20 | | 27 | 0,20 | | | |

Comme on peut le constater, certains composés parmi ceux synthétisés sont environ cent fois plus actifs que le GHB et présentent donc des propriétés sédatives améliorées.

Les composés de la présente invention sont donc particulièrement intéressants en ce qui concerne leur utilisation pour l'obtention d'un médicament destiné au traitement des affections neurologiques ou mentales dans lesquelles le système nerveux central joue un rôle. Il s'agit en particulier des affections dans lesquelles les récepteurs GHB sont impliqués et qui peuvent bénéficier des effets d'un agoniste ou d'un antagoniste de ces récepteurs GHB : régulation du sommeil et de la sécrétion d'hormones, en particulier d'hormones de croissance, diminution de l'anxiété ou augmentation de la vigilance, activité antiépileptique, régulation du poids et de la prise alimentaire, régulation de l'humeur ou activité antidépressive, activité neuroleptique, régulation du rythme circadien, activité hypnotique ou anesthésique, activité de neuroprotection ou anti-ischémique, activité dans la processus de sevrage aux drogues et dans l'addiction.

Ces médicaments sont **caractérisés en ce qu**'ils comprennent en tant que principe actif au moins un composé de formule générale I, I' ou I" telles que définies aux revendications 1 à 3.

De préférence, le ou les composés précités utilisés en tant que principe actif sont un ou plusieurs sels de sodium de formule générale I" obtenus par neutralisation d'un composé de formule générale I ou I' comportant une fonction acide pour le groupement W définis aux revendications 1 ou 2.

### 2) Essais in vivo : étude électroencéphalographique (EEG) des rats ayant reçu 2-4 mmoles/kg d'analogues de synthèse du GHB

Des rats mâles de souche Wistar, pesant 200 g au début de l'expérience, provenant du Centre d'Elevage Janvier (Route des Chênes-Secs, Le Genest St-Isie, 53940 France) sont utilisés pour cette étude.

Après huit jours d'adaptation aux conditions d'élevage de l'Animalerie Centrale de la Faculté de Médecine (11, rue Humann 67084 Strasbourg), ces animaux sont placés en cages individuelles (cages Makrolon type 3H de 425x266x150) dans un rythme jour/nuit standard de [7h/19h], l'eau et la nourriture (UAR réf. A04) étant à leur disposition permanente. Les animaux sont ensuite transférés dans un local d'expérimentation sur des supports pouvant accueillir 24 cages.

### a) Implantation d'électrodes corticales fronto-pariétales.

La procédure chirurgicale est la suivante : après un mois d'habituation au cycle jour/nuit [10h/22h], les rats sont pesés puis anesthésiés avec de la kétamine (Imalgène 500 Merial) à raison de 150 mg/kg i.p. Après avoir placé l'animal dans un cadre stéréotaxique (Narishige), une incision rostro-caudale est effectuée à l'aide d'un scalpel régulation du poids et de la prise alimentaire, régulation de l'humeur ou activité antidépressive, activité neuroleptique, régulation du rythme circadien, activité hypnotique ou anesthésique, activité de neuroprotection ou anti-ischémique, activité dans la processus de sevrage aux drogues et dans l'addiction.

Ces médicaments sont **caractérisés en ce qu**'ils comprennent en tant que principe actif au moins un composé de formule générale I, I' ou I" telles que définies aux revendications 1 à 3.

De préférence, le ou les composés précités utilisés en tant que principe actif sont un ou plusieurs sels de sodium de formule générale I" obtenus par neutralisation d'un composé de formule générale I ou I' comportant une fonction acide pour le groupement W définis aux revendications 1 ou 2.

### 2) Essais in vivo : étude électroencéphalographique (EEG) des rats ayant reçu 2-4 mmoles/kg d'analogues de synthèse du GHB

Des rats mâles de souche Wistar, pesant 200 g au début de l'expérience, provenant du Centre d'Elevage Janvier (Route des Chênes-Secs, Le Genest St-Isle, 53940 France) sont utilisés pour cette étude.

Après huit jours d'adaptation aux conditions d'élevage de l'Animalerie Centrale de la Faculté de Médecine (11, rue Humann 67084 Strasbourg), ces animaux sont placés en cages individuelles (cages Makrolon type 3H de 425x266x150) dans un rythme jour/nuit standard de [7h/19h], l'eau et la nourriture (UAR réf. A04) étant à leur disposition permanente. Les animaux sont ensuite transférés dans un local d'expérimentation sur des supports pouvant accueillir 24 cages.

### a) Implantation d'électrodes corticales fronto-pariétales.

La procédure chirurgicale est la suivante : après un mois d'habituation au cycle jour/nuit [10h/22h], les rats sont pesés puis anesthésiés avec de la kétamine (Imalgène 500 Merial) à raison de 150 mg/kg i.p. Après avoir placé l'animal dans un cadre stéréotaxique (Narishige), une incision rostro-caudale est effectuée à l'aide d'un scalpel stérilisé (lames n°3, Swann-Morton England).

Les sutures osseuses pariétales, Lambda et Bregma, sont mises à jour afin de servir de repère stéréotaxique (point 0). Après avoir perforé la boite crânienne à l'aide d'une fraise dentaire sans porter atteinte aux méninges (Minitor Narishige) deux vis inox de 500 µm de diamètre (Magister, 4 rue du Lac 25130 Villers Le Lac) sont implantées aux coordonnées suivantes: Bregma AP: ± 4 mm et ML: ± 3 mm.

Deux fils de cuivre sont soudés à l'aide d'étain aux vis d'une part et au connecteur femelle d'autre part (VP Electronic, Square de la Poterne, 91302 Massy Cedex). Le montage effectué est ensuite recouvert d'une résine polymérisante (Paladur, Kulzer, Allemagne).

Les rats implantés sont ensuite placés dans leurs cages respectives. Une période postopératoire supérieure à 48 heures est respectée avant tout enregistrement.

### b) Enregistrement EEG

Tous les enregistrements EEG sont effectués pendant les premières heures de la phase obscure (c'est-à-dire entre 10h et 13h) qui représente la période d'éveil et d'activité intense des animaux.

Les rats sont placés dans une cage en Plexiglas (170x170x300) et après une période d'habituation à leur nouvel environnement de 30 minutes, ils sont enregistrés en continu pendant une durée de 3 heures après injection i.p. (2 ml/kg) soit de NaCl à 0,9%, soit du ligand à étudier. Le tracé EEG est effectué à l'aide d'un enregistreur à 8 pistes (ALVAR Electronic, 6 rue du Progrès, 93511-Montreuil) avec une vitesse de défilement de 0,5 cm/s.

### c) Calcul de la durée du sommeil lent profond (SWS)

Les durées cumulées de sommeil lent profond de chaque animal sont évaluées par tranches de 20 minutes pour la durée totale de l'enregistrement de 3 heures (1 cm = 2 secondes).

### d) Analyse statistique et représentation graphique

La comparaison statistique est effectuée à l'aide d'un test d'analyse de variance (Anova) suivi d'un test de comparaisons multiples. Les animaux (6 à 8/groupe/dose) sont enregistrés à la fois sous NaCl à 0,9% (valeur témoin) et après administration du produit à étudier administré à la dose de 2 mmoles/kg.

Les résultats sont représentés en exprimant les moyennes ± SEM des durées cumulées en minutes de sommeil lent profond par tranches horaires de 20 minutes par rapport à l'affinité du ligand pour le récepteur GHB (CI₅₀) dans le tableau ci-après. Les chiffres entre parenthèses correspondent aux pourcentages de sommeil lent profond par rapport à la durée totale de sommeil.

**Tableau 4 : Résultats in vivo**

| N° du Composé testé | CI₅₀ µM | Dosage utilisé mmole/kg i.p. | Durée de sommeil lent profond (mn) | Augmentation de la durée du sommeil lent profond (mn) |
|---|---|---|---|---|
| NaCl | - | - | 11 ± 4 (7%) | - |
| 2 | 0,30 | 0,28 | 53±7 | 42 (23%) |

Comme on peut le constater, les composés selon l'invention permettent une augmentation significative de la durée du sommeil lent profond.

En conséquence, la présente invention a également pour objet une composition pharmaceutique comprenant à titre de principe actif au moins un composé de formule générale I, I' ou un sel de formule générale I" telles que décrites dans les revendications 1 à 3.

Les compositions pharmaceutiques revendiquées peuvent comprendre en outre d'autres excipients ou véhicules pharmaceutiquement acceptables.

La présente invention permet l'utilisation d'un composé conforme à ladite invention pour l'obtention d'un médicament comprenant en tant que principe actif au moins un composé de formule générale I, I' ou I" précitées pour le traitement d'une maladie pouvant être traitée par l'administration d'un agoniste ou antagoniste des récepteurs GHB, en particulier les affections neurologiques ou mentales du système nerveux central et notamment la régulation du sommeil et de la sécrétion d'hormones, en particulier d'hormones de croissance, la diminution de l'anxiété ou l'augmentation de la vigilance, l'activité antiépileptique, la régulation du poids et de la prise alimentaire, la régulation de l'humeur ou l'activité antidépressive, l'activité neuroleptique, la régulation du rythme circadien, l'activité hypnotique ou anesthésique, l'activité de neuroprotection ou anti-ischémique, l'activité dans la processus de sevrage aux drogues et dans l'addiction.

Grâce aux composés de la présente invention, il devient également possible de proposer un procédé de traitement d'une maladie chez un mammifère pouvant être traitée par l'administration d'un agoniste du GHB, en particulier les maladies du système nerveux central et notamment les maladies relatives au sommeil et à l'anxiété, ledit traitement comprenant l'administration audit mammifère d'une quantité efficace sur le plan thérapeutique d'au moins un composé de formule générale I, I' ou I" selon la présente invention, de préférence d'au moins un sel de sodium choisi parmi les composés de formule générale I", en particulier ceux mentionnés au tableau 2.

Les filtres sont ensuite « comptés » par scintillation liquide dans un compteur, en présence d'un liquide de scintillation. Les résultats sont exprimés en pourcentage de la fixation réversible totale déterminée en présence d'un excès de GHB non radioactif (500 µM, 60-80% de la fixation totale). Les analyses statistiques des CI₅₀ (concentration d'analogues synthétiques susceptibles de déplacer 50% de la fixation réversible du ³H= GHB) sont résumées dans le tableau ci-après. Plus le CI₅₀ est faible, plus l'affinité du ligand pour le récepteur est forte. Les analyses statistiques des courbes de déplacement sont réalisées à l'aide du logiciel GraphPad Prism (San Diego, CA).

Les résultats obtenus sont regroupés dans le tableau suivant :

**Tableau 3 : Résultats in vitro**

| | | | | | | | |
|---|---|---|---|---|---|---|---|
| N° du composé | CI₅₀ µM | | N° du composé | CI₅₀ µM | | N° du composé | CI₅₀ µM |
| GHB | 5,60 | | 15 | 0,70 | | 30 | 0,50 |
| 1 | 6,80 | | 16 | 0,20 | | 31 | 0,10 |
| 2 | 0,30 | | 17 | 1,40 | | 32 | 0,30 |
| 3 | 0,30 | | 18 | 0,10 | | 33 | 0,20 |
| 4 | 1,60 | | 19 | 1,10 | | 34 | 23,5 |
| 5 | 3,90 | | 20 | 24,7 | | 35 | 2,30 |
| 6 | 2,50 | | 21 | 16,2 | | 36 | 1,80 |
| 7 | 1,80 | | 22 | 1,20 | | 37 | 25,0 |
| 8 | 0,80 | | 23 | 0,20 | | 38 | 0,80 |
| 9 | 2,30 | | 24 | 0,08 | | 39 | 34,2 |
| 10 | 0,60 | | 25 | 0,1 | | 40 | 1,10 |
| 11 | 0,90 | | 26 | 0,30 | | 41 | 3,80 |
| 12 | 0,60 | | 27 | 0,20 | | 42 | 0,60 |
| 13 | 0,10 | | 28 | 0,10 | | | |
| 14 | 0,30 | | 29 | 0,90 | | | |

Comme on peut le constater, certains composés parmi ceux synthétisés sont environ cent fois plus actifs que le GHB et présentent donc des propriétés sédatives améliorées.

## Revendications

1. Composés de formule générale 1 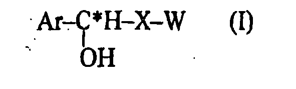 dans laquelle Ar représente l'un des mono, bi ou tricycles suivants : 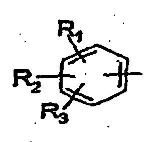 dans lequel:
- R₁, représente indépendamment un groupement alkyle en C2 à C6, un groupement aralkyle, un groupement hydroxyle, un groupement méthoxy, un groupement COOEt, un groupement NHCOCH₃, un groupement NH₂ ou un groupement CON(CH₃)₂,
- R₂ représente indépendamment un halogène, un groupement alkyle, un groupement aralkyle, un groupement hydroxyle, un groupement méthoxy, un groupement COOET, un groupement NHCOCH₃, un groupement NH₂, ou un groupement CON(CR₃)₂, et
- R₃ représente l'hydrogène, 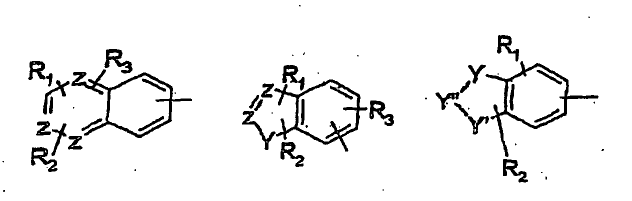 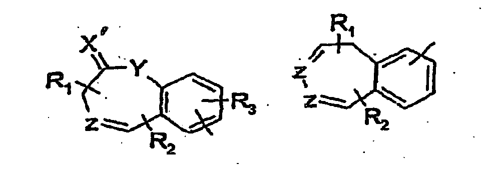 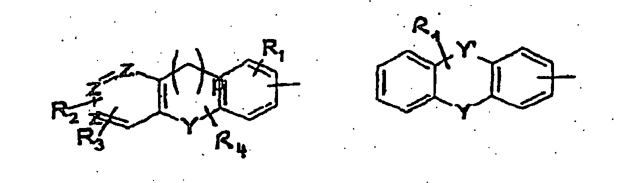
dans lesquels :
- R₁, R₂, R₃ et R₄ représentent indépendamment un atome d'hydrogène, un halogène, un groupement alkyle, un groupement aryle, un groupement aralkyle, un groupement hydroxyle, un groupement méthoxy, un groupement acétyle, un groupement tosyle, un groupement COOEt, un groupement NHCOCH₃, un groupement NH₂, un groupement CON(CH₃)₂ ou un groupement NO₂,
- chaque z représente indépendamment un atome d'azote ou de carbone,
- Y et Y' représentent indépendamment un atome de carbone, de soufre, d'oxygène ou un atome d'azote,
- Y" représente un groupement méthylène, éthylène ou propylène,
- X' représente indépendamment un atome de soufre ou d'oxygène,
- p vaut 0, 1 ou 2,
dans laquelle X représente indépendamment (CH₂)₂ ou (CH₂)₃ et, dans laquelle W représente COOH, COO⁻M⁺, (M⁺ représentant un contre-ion acceptable sur le plan pharmaceutique), CH₂OH, COOR (avec R représentant un groupement alkyle), SO₃H ou PO₃H₂ ou un groupement choisi parmi les suivants : 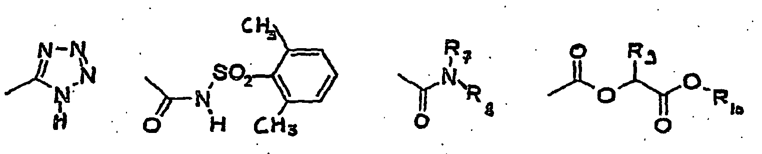
dans lesquels R₇ et R₈ représentent indépendamment un atome d'hydrogène, un groupement alkyle, un groupement aryle, un groupement aralkyle ou un groupement hydroxyle,
dans lesquels R₉ représente indépendamment un atome d'hydrogène ou un groupement méthyle et dans lesquels R₁₀ représente indépendamment un groupement éthyle, C₁₂H₁₅ ou adamantyle.

2. Composés selon la revendication 1 de formule générale I' : 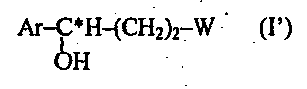 dans laquelle W représente COOH ou COO⁻M⁺, (M⁺ représentant un contre-ion acceptable sur le pian pharmaceutique) et dans laquelle Ar est choisi parmi les groupes définis à la revendication 1.

3. Composés selon la revendication 1 ou 2, **caractérisé en ce qu'**il s'agit des sels de formule générale I" 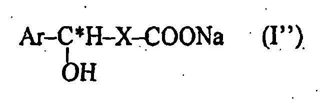 dans laquelle Ar et X sont tels que définis à la revendication 1 ou 2.

4. Composé selon la revendication 3, **caractérisé en ce qu'**il s'agit du sel de sodium de l'acide 4-(3,4-diméthoxyphényl)-4-hydroxybutanoïque.

5. Composé selon la revendication 3, **caractérisé en ce qu'**il s'agit du sel de sodium de l'acide 4-[4-(acétylamino)phényl]-4-hydroxybutanoïque.

6. Composé selon la revendication 3, **caractérisé en ce qu'**il s'agit du sel de sodium de l'acide 4-(2,3-dihydro-1-benzofuran-5-yl)-4-hydroxybutanoïque.

7. Composé selon la revendication 3, **caractérisé en ce qu'**il s'agit du sel de sodium de l'acide 4-(1-acétyl-2,3-dihydro-1*H*-indol-5-yl)-4-hydroxybutanoïque.

8. Composé selon la revendication 3, **caractérisé en ce qu'**il s'agit du sel de sodium de l'acide 4-(2,3-dihydro-1*H*-inden-5-yl)-4-hydroxy butanoïque.

9. Composé selon la revendication 3, **caractérisé en ce qu'**il s'agit du sel de sodium de l'acide 4-[2-(éthoxycarbonyl)-1*H-*indol-5-yl]-4-hydroxybutanoïque.

10. Composé selon la revendication 3, **caractérisé en ce qu'**il s'agit du sel de sodium de l'acide 4-hydroxy-4-(1*H*-indol-5-yl)-butanoïque.

11. Composé selon la revendication 3, **caractérisé en ce qu'**il s'agit du sel de sodium de l'acide 4-hydroxy-4-(4-oxo-1,4-dihydroquinolin-7-yl)-butanoïque.

12. Composé selon la revendication 3, **caractérisé en ce qu'**il s'agit du sel de sodium de l'acide 4-hydroxy-4-(4-oxo-1,4-dihydroq'uinolin-6-yl)-butanoïque.

13. Composé selon la revendication 3, **caractérisé en ce qu'**il s'agit du sel de sodium de l'acide 4-hydroxy-4-(2-oxo-2,3-dihydro-1*H-*indol-5-yl)-butanoïque.

14. Composé selon la revendication 3, **caractérisé en ce qu'**il s'agit du sel de sodium de l'acide 4-{2-[(diméthylamino)carbonyl]-1*H-*indol-5-yl}-4-hydroxybutanoïque.

15. Composé selon la revendication 3, **caractérisé en ce qu'**il s'agit du sel de sodium de l'acide 4-dibenzo[b,d]thiophène-2-yl-4-hydroxybutanoïque.

16. Composé selon la revendication 3, **caractérisé en ce qu'**il s'agit du sel de sodium de l'acide 4-dibenzo[b,d]furan-2-yl-4-hydroxybutanoïque,

17. Composé selon la revendication 3, **caractérisé en ce qu'**il s'agit du sel de sodium de l'acide 4-(9-acétyl-9*H*-carbazol-3-yl-4-hydroxybutanoïque.

18. Composé selon la revendication 3, **caractérisé en ce qu'**il s'agit du sel de sodium de l'acide 4-hydroxy-4-phénoxathiin-2-yl)butanoïque.

19. Composé selon la revendication 3, **caractérisé en ce qu'**il s'agit du sel de sodium de l'acide 4-hydroxy-4-(phénoxathiin-3-yl)butanoïque.

20. Composé selon la revendication 3, **caractérisé en ce qu'**il s'agit du sel de sodium de l'acide 4-(5-acétyl-10, 11-dihydro-5*H-*dibenzo[b,f]azépin-3-yl)-4-hydroxybutanoïque.

21. Composé selon la revendication 3, **caractérisé en ce qu'**il s'agit du sel de sodium de l'acide 4-hydroxy-4-(9*H*-xanthen-2-yl)butanoïque.

22. Composé selon la revendication 3, **caractérisé en ce qu'**sil s'agit du sel de sodium de l'acide 4-(9*H-*fluoren-3-yl)hydroxybutanoïque.

23. Composé selon la revendication 3, **caractérisé en ce qu'**il s'agit du sel de sodium de l'acide 4-hydrow-4-(4-oxo-4,5-dihydro-3*H-*pyridazo(4,5-b)indol-8-yl)butanoïque.

24. Composé selon la revendication 3, **caractérisé en ce qu'**il s'agit du sel de sodium de l'acide 4-hydroxy-4-(4-oxo-4,5-dihydro-3*H* pyridazo(4,5-b)indol-6-yl)butanoïque.

25. Composé selon la revendication 3, **caractérisé en ce qu'**il s'agit du sel de sodium de l'acide 4-(3,3-diméthyl-2-oxo-1,2,3,4-tétrahydroquinolin-6-yl)-4-hydroxybutanoïque.

26. Utilisation d'un composé selon l'une quelconque des revendications 1 à 25 pour l'obtention d'un médicament comprenant en tant que principe actif au moins un composé de formule générale I, I' ou I" telles que définies dans lesdites revendications 1 à 3 pour le traitement d'une maladie pouvant être traitée par l'administration d'un agoniste ou antagoniste des récepteurs GHB, en particulier les affections neurologiques ou mentales du système nerveux central et notamment la régulation du sommeil et de la sécrétion d'hormones, en particulier d'hormones de croissance, la diminution de l'anxiété ou l'augmentation de la vigilance, l'activité antiépileptique, la régulation du poids et de la prise alimentaire, la régulation de l'humeur ou l'activité antidépressive, l'activité neuroleptique, la régulation du rythme circadien, l'activité hypnotique ou anesthésique, l'activité de neuroprotection ou anti-ischémique, l'activité dans la processus de sevrage aux drogues et dans l'addiction.

27. Utilisation selon la revendication 26, **caractérisée en ce que** le ou les composés utilisés en tant que principe actif sont un ou plusieurs sels de sodium de la formule générale I" de la revendication 3 obtenus par neutralisation d'un composé de formule générale I ou I' des revendications 1 ou 2 et comportant une fonction acide pour le groupement W.

28. Composition pharmaceutique **caractérisée en ce qu'**elle comprend à titre de principe actif au moins un composé de formule générale I, I' ou I" telles que définies aux revendications 1 à 3.

## Claims

1. Compounds of general formula I 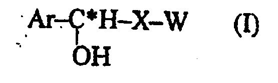 in which formula Ar represents one of the following monocycles, bicycles or tricycles: 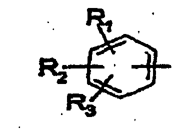 in which:
- R₁ independently represents a C₂ to C₆ alkyl group, an aralkyl group, a hydroxyl group, a methoxy group, a COOEt group, an NHCOCH₃ group, an NH₂ group or a CON(CH₃)₂ group,
- R₂ independently represents a halogen, an alkyl group, an aralkyl group, a hydroxyl group, a methoxy group, a COOEt group, an NHCOCH₃ group, an NH₂ group or a CON(CH₃)₂ group, and
- R₃ represents hydrogen; 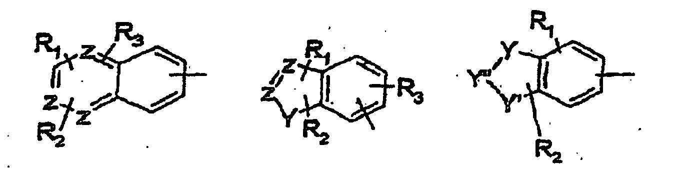 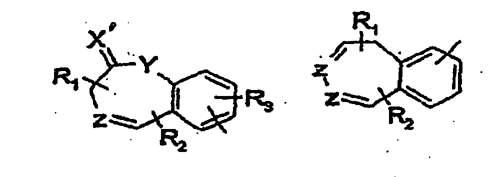 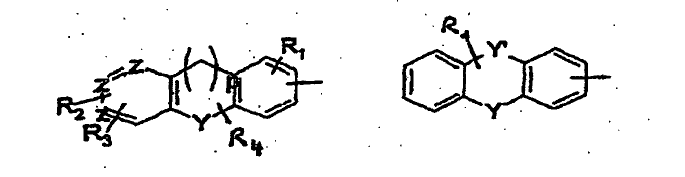
in which:
- R₁, R₂, R₃ and R₄ independently represent a hydrogen atom, a halogen, an alkyl group, an aryl group, an aralkyl group, a hydroxyl group, a methoxy group, an acetyl group, a tosyl group, a COOEt group, an NHCOCH₃ group, an NH₂ group, a CON(CH₃)₂ group or an NO₂ group,
- each z independently represents a nitrogen or carbon atom,
- Y and Y' independently represent a carbon, sulphur, oxygen or nitrogen atom,
- Y" represents a methylene, ethylene or propylene group,
- X' independently represents a sulphur or oxygen atom,
- p is 0, 1 or 2,
in which formula X independently represents (CH₂)₂ or (CH₂)₃, and in which formula W represents COOH, COO⁻M⁺ (M⁺ representing a pharmaceutically acceptable counterion), CH₂OH, COOR (with R representing an alkyl group), SO₃H or PO₃H₂ or a group chosen from the following: 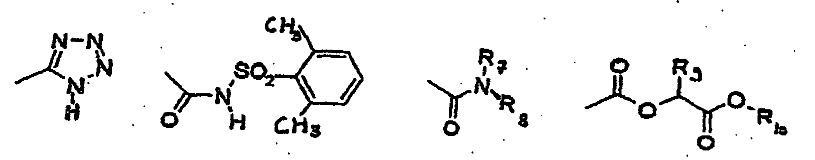
in which R₇ and R₈ independently represent a hydrogen atom, an alkyl group, an aryl group, an aralkyl group or a hydroxyl group,
in which R₉ independently represents a hydrogen atom or a methyl group and in which R₁₀ independently represents an ethyl, C₁₂H₁₅ or adamantyl group.

2. Compounds according to Claim 1, of general formula I' : 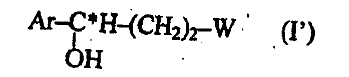 in which formula W represents COOH or COO⁻M⁺ (M⁺ representing a pharmaceutically acceptable counterion) and in which formula Ar is chosen from the groups defined in Claim 1.

3. Compounds according to Claim 1 or 2, **characterized in that** they are the salts of general formula I" 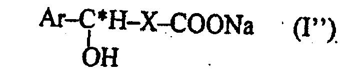 in which formula Ar and X are as defined in Claim 1 or 2.

4. Compound according to Claim 3, **characterized in that** it is the sodium salt of 4-(3,4-dimethoxyphenyl)-4-hydroxybutanoic acid.

5. Compound according to Claim 3, **characterized in that** it is the sodium salt of 4-[4-(acetylamino)phenyl]-4-hydroxybutanoic acid.

6. Compound according to Claim 3, **characterized in that** it is the sodium salt of 4-(2,3-dihydro-1-benzofuran-5-yl)-4-hydroxybutanoic acid.

7. Compound according to Claim 3, **characterized in that** it is the sodium salt of 4-(1-acetyl-2,3-dihydro-1*H*-indol-5-yl)-4-hydroxybutanoic acid.

8. Compound according to Claim 3, **characterized in that** it is the sodium salt of 4-(2,3-dihydro-1*H*-inden-5-yl)-4-hydroxybutanoic acid.

9. Compound according to Claim 3, **characterized in that** it is the sodium salt of 4-[2-(ethoxycarbonyl)-1*H-*indol-5-yl]-4-hydroxybutanoic acid.

10. Compound according to Claim 3, **characterized in that** it is the sodium salt of 4-hydroxy-4-(1*H*-indol-5-yl)butanoic acid.

11. Compound according to Claim 3, **characterized in that** it is the sodium salt of 4-hydroxy-4-(4-oxo-1,4-dihydroquinolin-7-yl)butanoic acid.

12. Compound according to Claim 3, **characterized in that** it is the sodium salt of 4-hydroxy-4-(4-oxo-1,4-dihydroquinolin-6-yl)butanoic acid.

13. Compound according to Claim 3, **characterized in that** it is the sodium salt of 4-hydroxy-4-(2-oxo-2,3-dihydro-1*H*-indol-5-yl)butanoic acid.

14. Compound according to Claim 3, **characterized in that** it is the sodium salt of 4-{2-[(dimethylamino)carbonyl]-1*H*-indol-5-yl}-4-hydroxybutanoic acid.

15. Compound according to Claim 3, **characterized in that** it is the sodium salt of 4-dibenzo[b,d]thiophen-2-yl-4-hydroxybutanoic acid.

16. Compound according to Claim 3, **characterized in that** it is the sodium salt of 4-dibenzo[b,d]furan-2-yl-4-hydroxybutanoic acid.

17. Compound according to Claim 3, **characterized in that** it is the sodium salt of 4-(9-acetyl-9*H*-carbazol-3-yl)-4-hydroxybutanoic acid.

18. Compound according to Claim 3, **characterized in that** it is the sodium salt of 4-hydroxy-4-(phenoxathiin-2-yl)butanoic acid.

19. Compound according to Claim 3, **characterized in that** it is the sodium salt of 4-hydroxy-4-(phenoxathiin-3-yl)butanoic acid.

20. Compound according to Claim 3, **characterized in that** it is the sodium salt of 4-(5-acetyl-10,11-dihydro-5*H*-dibenzo[b,f]azepin-3-yl)-4-hydroxy-butanoic acid.

21. Compound according to Claim 3, **characterized in that** it is the sodium salt of 4-hydroxy-4-(9*H*-xanthen-2-yl)butanoic acid.

22. Compound according to Claim 3, **characterized in that** it is the sodium salt of 4-(9*H*-fluoren-3-yl)hydroxybutanoic acid.

23. Compound according to Claim 3, **characterized in that** it is the sodium salt of 4-hydroxy-4-(4-oxo-4,5-dihydro-3*H*-pyridazo(4,5-b)indol-8-yl)butanoic acid.

24. Compound according to Claim 3, **characterized in that** it is the sodium salt of 4-hydroxy-4-(4-oxo-4,5-dihydro-3*H*-pyridazo(4,5-b)indol-6-yl)butanoic acid.

25. Compound according to Claim 3, **characterized in that** it is the sodium salt of 4-(3,3-dimethyl-2-oxo-1,2,3,4-tetrahydroquinolin-6-yl)-4-hydroxybutanoic acid.

26. Use of a compound according to any one of Claims 1 to 25, for producing a medicament comprising, as active ingredient, at least one compound of general formula I, I' or I" as defined in said Claims 1 to 3, for the treatment of a disease which can be treated by the administration of a GHB receptor agonist or antagonist, in particular neurological or mental conditions of the central nervous system, and especially sleep regulation and the regulation of hormone secretion, in particular the secretion of growth hormones, reducing anxiety or increasing vigilance, antiepileptic activity, regulation of weight and of food intake, regulation of mood or antidepressive activity, neuroleptic activity, regulation of the circadian rhythm, hypnotic or anaesthetic activity, neuroprotective or anti-ischemic activity, and activity in the drug withdrawal process and in addiction.

27. Use according to Claim 26, **characterized in that** the compound(s) used as active ingredient is (are) one or more sodium salts of general formula I" of Claim 3, obtained by neutralization of a compound of general formula I or I' of Claim 1 or 2, and comprising an acid function for the group W.

28. Pharmaceutical composition, **characterized in that** it comprises, as active ingredient, at least one compound of general formula I, I' or I" as defined in Claims 1 to 3.

## Patentansprüche

1. Verbindungen der allgemeinen Formel 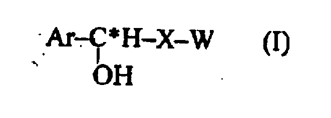 in der Ar einen der folgenden Mono-, Bi- oder Tricyclen darstellt: 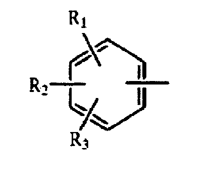 wobei:
R₁ unabhängig eine C₂- bis C₆-Alkylgruppe, eine Aralkylgruppe, eine Hydroxylgruppe, eine Methoxygruppe, eine COOEt-Gruppe, eine NHCOCH₃-Gruppe, eine NH₂-Gruppe oder eine CON(CH₃)₂-Gruppe darstellt,
R₂ unabhängig ein Halogen, eine Alkylgruppe, eine Aralkylgruppe, eine Hydroxylgruppe, eine Methoxygruppe, eine COOEt-Gruppe, eine NHCOCH₃-Gruppe, eine NH₂-Gruppe oder eine CON(CH₃)₂-Gruppe darstellt und
R₃ Wasserstoff, 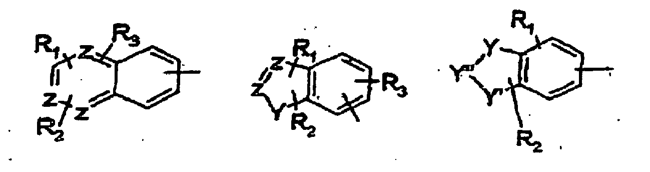 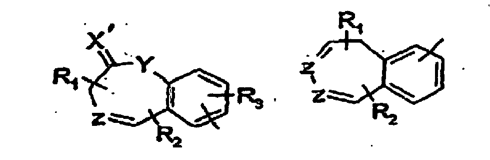 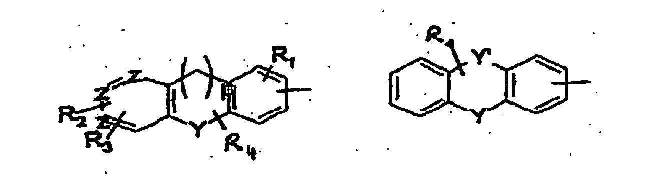
wobei R₁, R₂, R₃ und R₄ unabhängig ein Wasserstoffatom, ein Halogen, eine Alkylgruppe, eine Arylgruppe, eine Aralkylgruppe, eine Hydroxylgruppe, eine Methoxygruppe, eine Acetylgruppe, eine Tosylgruppe, eine COOEt-Gruppe, eine NHCOCH₃-Gruppe, eine NH₂-Gruppe, eine CON(CH₃)₂-Gruppe oder eine NO₂-Gruppe darstellen,
jedes z unabhängig ein Stickstoffatom oder ein Kohlenstoffatom darstellt,
Y und Y' unabhängig ein Kohlenstoffatom, ein Schwefelatom, ein Sauerstoffatom oder ein Stickstoffatom darstellen,
Y" eine Methylengruppe, eine Ethylengruppe oder eine Propylengruppe darstellt,
X' unabhängig ein Schwefelatom oder ein Sauerstoffatom darstellt,
p 0, 1 oder 2 ist,
darstellt,
wobei X unabhängig (CH₂)₂ oder (CH₂)₃ darstellt und wobei
W COOH, COO⁻M⁺ (M⁺ stellt ein pharmazeutisch akzeptables Gegenion dar), CH₂OH, COOR (wobei R eine Alkylgruppe darstellt), SO₃H oder PO₃H₂ oder eine Gruppe ausgewählt aus den folgenden Gruppen: 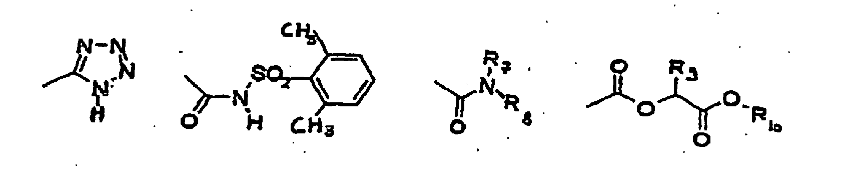
wobei R₇ und R₈ unabhängig ein Wasserstoffatom eine Alkylgruppe, eine Arylgruppe, eine Aralkylgruppe oder eine Hydroxylgruppe darstellen,
wobei R₉ unabhängig ein Wasserstoffatom oder eine Methylgruppe darstellt und wobei R₁₀ unabhängig eine Ethylgruppe, eine C₁₂H₁₅-Gruppe oder eine Adamantylgruppe darstellt, darstellt.

2. Verbindungen gemäß Anspruch 1 der allgemeinen Formel I': 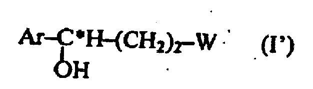 wobei W COOH oder COO⁻M⁺ (M⁺ stellt ein pharmazeutisch akzeptables Gegenion dar) darstellt und wobei Ar aus den Gruppen wie in Anspruch 1 dargestellt ausgewählt ist.

3. Verbindungen gemäß Anspruch 1 oder 2, **dadurch gekennzeichnet, dass** es sich um Salze mit der allgemeinen Formel I" handelt 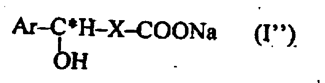 wobei Ar, X und n wie in den Ansprüchen 1 oder 2 definiert sind.

4. Eine Verbindung gemäß Anspruch 3, **dadurch gekennzeichnet, dass** es sich um das Natriumsalz von 4-(3,4-Dimethoxyphenyl)-4-hydroxybutansäure handelt.

5. Eine Verbindung gemäß Anspruch 3, **dadurch gekennzeichnet, dass** es sich um das Natriumsalz von 4-[4-(Acetylamino)phenyl]-4-hydroxybutansäure handelt.

6. Eine Verbindung gemäß Anspruch 3, **dadurch gekennzeichnet, dass** es sich um das Natriumsalz von 4-(2,3-Dihydro-1-benzofuran-5-yl)-4-hydroxybutansäure handelt.

7. Eine Verbindung gemäß Anspruch 3, **dadurch gekennzeichnet, dass** es sich um das Natriumsalz von 4-(1-Acetyl-2,3-dihydro-1*H*-indol-5-yl)-4-hydroxybutansäure handelt.

8. Eine Verbindung gemäß Anspruch 3, **dadurch gekennzeichnet, dass** es sich um das Natriumsalz von 4-(2,3-Dihydro-1*H*-inden-5-yl)-4-hydroxybutansäure handelt.

9. Eine Verbindung gemäß Anspruch 3, **dadurch gekennzeichnet, dass** es sich um das Natriumsalz von 4-[2-(Ethoxycarbonyl)-1*H*-indol-5-yl]-4-hydroxybutansäure handelt.

10. Eine Verbindung gemäß Anspruch 3, **dadurch gekennzeichnet, dass** es sich um das Natriumsalz von 4-Hydroxy-4-(1*H*-indol-5-yl)butansäure handelt.

11. Eine Verbindung gemäß Anspruch 3, **dadurch gekennzeichnet, dass** es sich um das Natriumsalz von 4-Hydroxy-4-(4-oxo-1,4-dihydrochinolin-7-yl)butansäure handelt.

12. Eine Verbindung gemäß Anspruch 3, **dadurch gekennzeichnet, dass** es sich um das Natriumsalz von 4-Hydroxy-4-(4-oxo-1,4-dihydrochinolin-6-yl)butansäure handelt.

13. Eine Verbindung gemäß Anspruch 3, **dadurch gekennzeichnet, dass** es sich um das Natriumsalz von 4-Hydroxy-4-(2-oxo-2,3-dihydro-1*H*-indol-5-yl)butansäure handelt.

14. Eine Verbindung gemäß Anspruch 3, **dadurch gekennzeichnet, dass** es sich um das Natriumsalz von 4-{2-[(Dimethylamino)carbonyl]-1*H*-indol-5-yl}-4-hydroxybutansäure handelt.

15. Eine Verbindung gemäß Anspruch 3, **dadurch gekennzeichnet, dass** es sich um das Natriumsalz von 4-Dibenzo[b,d]thiophen-2-yl-4-hydroxybutansäure handelt.

16. Eine Verbindung gemäß Anspruch 3, **dadurch gekennzeichnet, dass** es sich um das Natriumsalz von 4-Dibenzo[b,d]furan-2-yl-4-hydroxybutansäure handelt.

17. Eine Verbindung gemäß Anspruch 3, **dadurch gekennzeichnet, dass** es sich um das Natriumsalz von 4-(9-Acetyl-9*H*-carbazol-3-yl-4-hydroxybutansäure handelt.

18. Eine Verbindung gemäß Anspruch 3, **dadurch gekennzeichnet, dass** es sich um das Natriumsalz von 4-Hydroxy-4-(phenoxathiin-2-yl)butansäure handelt.

19. Eine Verbindung gemäß Anspruch 3, **dadurch gekennzeichnet, dass** es sich um das Natriumsalz von 4-Hydroxy-4-(phenoxathiin-3-yl)butansäure handelt.

20. Eine Verbindung gemäß Anspruch 3, **dadurch gekennzeichnet, dass** es sich um das Natriumsalz von 4-(5-Acetyl-10,11-dihydro-5*H*-dibenzo[b,f]azepin-3-yl)-4-hydroxybutansäure handelt.

21. Eine Verbindung gemäß Anspruch 3, **dadurch gekennzeichnet, dass** es sich um das Natriumsalz von 4-Hydroxy-4-(9*H*-xanthen-2-yl)butansäure handelt.

22. Eine Verbindung gemäß Anspruch 3, **dadurch gekennzeichnet, dass** es sich um das Natriumsalz von 4-(9H-Fluoren-3-yl)hydroxybutansäure handelt.

23. Eine Verbindung gemäß Anspruch 3, **dadurch gekennzeichnet, dass** es sich um das Natriumsalz von 4-Hydroxy-4-(4-oxo-4,5-dihydro-3H-pyridazo(4,5-b)indol-8-yl)butansäure handelt.

24. Eine Verbindung gemäß Anspruch 3, **dadurch gekennzeichnet, dass** es sich um das Natriumsalz von 4-Hydroxy-4-(4-oxo-4,5-dihydro-3*H*-pyridazo(4,5-b)indol-6-yl)butansäure handelt.

25. Eine Verbindung gemäß Anspruch 3, **dadurch gekennzeichnet, dass** es sich um das Natriumsalz von 4-(3,3-Dimethyl-2-oxo-1,2,3,4-tetrahydrochinolin-6-yl)-4-hydroxybutansäure handelt.

26. Verwendung einer Verbindung gemäß einem der Ansprüche 1 bis 25 zur Herstellung eines Medikamentes, welches als aktiven Inhaltsstoff mindestens eine Verbindung mit der allgemeinen Formel I, I' oder I", welche wie in den Ansprüchen 1 bis 3 definiert sind, enthält, zur Behandlung einer Krankheit, welche mittels Verabreichung eines Agonisten oder Antagonisten von GHB-Rezeptoren behandelt werden kann, insbesondere neurologische oder mentale Störungen des zentralen Nervensystems und insbesondere die Regulierung des Schlafs und die Sekretion von Hormonen, insbesondere von Wachstumshormonen, die Reduzierung von Angst oder die Erhöhung der Vigilanz, antiepileptische Wirkung, die Steuerung des Gewichtes und der Nahrungsaufnahme, die Steuerung der Stimmung oder antidepressive Wirkung, neuroleptische Wirkung, die Steuerung des zirkadianen Rhythmusses, hypnotische oder anästhesierende Wirkung, neuroschützende oder anti-ischämische Wirkung, Wirkung im Prozess des Drogenentzugs und der Abhängigkeit.

27. Verwendung gemäß Anspruch 26, **dadurch gekennzeichnet, dass** die Verbindung(en), die als aktive(r) Inhaltsstoff(e) eingesetzt wird/werden, ein oder mehrere Natriumsalze der allgemeinen Formel I" sind, die durch Neutralisation einer Verbindung der allgemeinen Formel I oder I' gemäß Anspruch 1 oder 2 erhalten wurden und eine Säurefunktion für die Gruppe W umfassen.

28. Pharmazeutische Zusammensetzung **dadurch gekennzeichnet, dass** sie als aktiven Inhaltsstoff mindestens eine Verbindung mit der allgemeinen Formel I, I' oder I" gemäß einem der Ansprüche 1 bis 3 umfasst.
